(19) Europäisches Patentamt
European Patent Office
Office européen des brevets

(11) **EP 2 107 470 B1**

(12) **EUROPEAN PATENT SPECIFICATION**

(45) Date of publication and mention
of the grant of the patent:
**30.08.2023 Bulletin 2023/35**

(21) Application number: **08016729.9**

(22) Date of filing: **24.09.2008**

(51) International Patent Classification (IPC):
**G06F 17/18** (2006.01)     **C12Q 1/686** (2018.01)

(52) Cooperative Patent Classification (CPC):
(C-Sets available)
**G06F 17/18; C12Q 1/686**          (Cont.)

(54) **PCR elbow determination using quadratic test for curvature analysis of a double sigmoid**

Bestimmung des Wendepunktes eines PCT-Graphen mittels quadratischem Test zur Kurvenanalyse einer Doppelsigmoidfunktion

Détermination du point d'inflexion d'un graphe PCR utilisant un test quadratique pour une analyse de courbe d'un double sigmoïde

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **25.09.2007 US 861188**

(43) Date of publication of application:
**07.10.2009 Bulletin 2009/41**

(73) Proprietors:
• **Roche Diagnostics GmbH**
**68305 Mannheim (DE)**
Designated Contracting States:
**DE**
• **F. Hoffmann-La Roche AG**
**4070 Basel (CH)**
Designated Contracting States:
**AT BE BG CH CY CZ DK EE ES FI FR GB GR HR HU IE IS IT LI LT LU LV MC MT NL NO PL PT RO SE SI SK TR**

(72) Inventors:
• **Aditya, Sane**
**Pleasanton,**
**CA 94566 (US)**
• **Kurnik, Roland T.**
**Foster City,**
**CA 94404 (US)**

(56) References cited:
**EP-A1- 1 798 650     EP-A2- 1 335 028**
**EP-A2- 1 804 172     US-A1- 2006 009 916**

(52) Cooperative Patent Classification (CPC): (Cont.)

C-Sets
**C12Q 1/686, C12Q 2545/114**

**Description**

BACKGROUND OF THE INVENTION

The present invention relates generally to systems and methods for determining the cycle threshold (Ct) value of a growth curve for a Polymerase Chain Reaction (PCR).

In particular, the present invention relates to determining whether the data for a growth curve represents or exhibits valid or significant growth, and if so determining the cycle threshold (Ct) value of a growth curve for the Polymerase Chain Reaction curve.

The Polymerase Chain Reaction (PCR) is an *in vitro* method for enzymatically synthesizing or amplifying defined nucleic acid sequences. The reaction typically uses two oligonucleotide primers that hybridize to opposite strands and flank a template or target DNA sequence that is to be amplified. Elongation of the primers is catalyzed by a heat-stable DNA polymerase. A repetitive series of cycles involving template denaturation, primer annealing, and extension of the annealed primers by the polymerase results in an exponential accumulation of a specific DNA fragment. Fluorescent probes or markers are typically used in the process to facilitate detection and quantification of the amplification process.

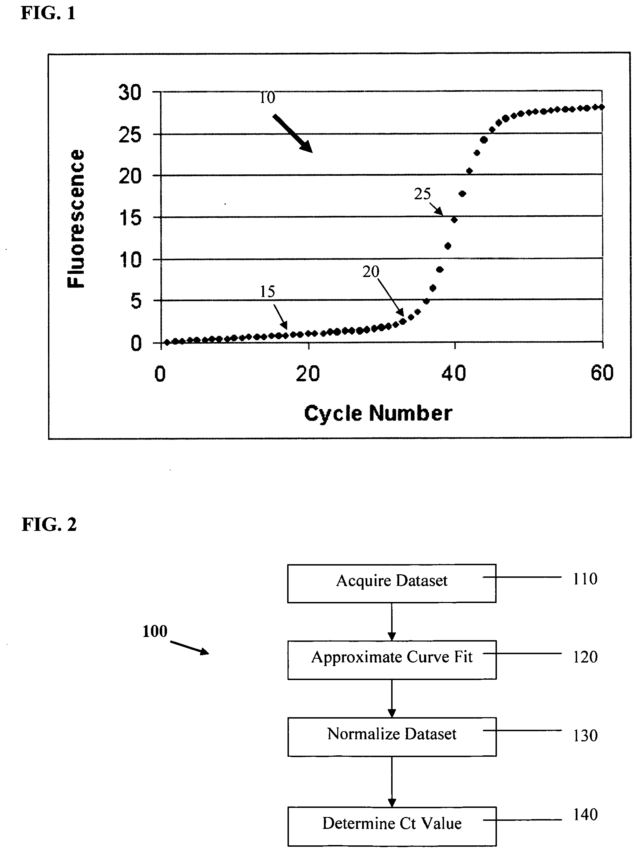

A typical real-time PCR curve is shown in FIG. 1, where fluorescence intensity values are plotted vs. cycle number for a typical PCR process. In this case, the formation of PCR products is monitored in each cycle of the PCR process. The amplification is usually measured in thermocyclers which include components and devices for measuring fluorescence signals during the amplification reaction. An example of such a thermocycler is the Roche Diagnostics LightCycler (Cat. No. 20110468). The amplification products are, for example, detected by means of fluorescent labeled hybridization probes which only emit fluorescence signals when they are bound to the target nucleic acid or in certain cases also by means of fluorescent dyes that bind to double-stranded DNA.

For a typical PCR curve, identifying a transition point at the end of the baseline region, which is referred to commonly as the elbow value or cycle threshold (Ct) value, is extremely useful for understanding characteristics of the PCR amplification process. The Ct value may be used as a measure of efficiency of the PCR process. For example, typically a defined signal threshold is determined for all reactions to be analyzed and the number of cycles (Ct) required to reach this threshold value is determined for the target nucleic acid as well as for reference nucleic acids such as a standard or housekeeping gene. The absolute or relative copy numbers of the target molecule can be determined on the basis of the Ct values obtained for the target nucleic acid and the reference nucleic acid (Gibson et al., Genome Research 6:995-1001; Bieche et al., Cancer Research 59:2759-2765, 1999; WO 97/46707; WO 97/46712; WO 97/46714). The elbow value in region 20 at the end of the baseline region 15 in FIG. 1 would be in the region of cycle number 30.

The elbow value in a PCR curve can be determined using several existing methods. For example, various current methods determine the actual value of the elbow as the value where the fluorescence reaches a predetermined level called the AFL (arbitrary fluorescence value). Other current methods might use the cycle number where the second derivative of fluorescence vs. cycle number reaches a maximum. All of these methods have drawbacks. For example, some methods are very sensitive to outlier (noisy) data, and the AFL value approach does not work well for data sets with high baselines. Traditional methods to determine the baseline stop (or end of the baseline) for the growth curve shown in FIG. 1 may not work satisfactorily, especially in a high titer situation. Furthermore, these algorithms typically have many parameters (e.g., 50 or more) that are poorly defined, linearly dependent, and often very difficult, if not impossible, to optimize.

EP1798650 and EP1804172 disclose PCR systems programmed to determine a cycle threshold (Ct) value. However, none of these documents discloses determining the cycle threshold (Ct) value including the determination of whether the PCR curve exhibits significant growth. EP1335028 discloses a PCR system that comprises fitting line to the whole PCR curve and computing a statistical significance of the linear fit. However, EP 1335028 does not disclose calculating an approximation function that fits the data set by applying a regression process, wherein the approximation function is a double sigmoid function as provided in the instant disclosure. Therefore it is desirable to provide systems and methods for determining the elbow value in curves, such as sigmoid-type or growth curves, and PCR curves in particular, which overcome the above and other problems. It is also desirable to determine, initially, whether the curves exhibit valid growth or whether the data should be discarded prior to consuming processing resources.

BRIEF SUMMARY OF THE INVENTION

The present invention provides novel, efficient systems and methods for determining whether the data for a growth curve represents or exhibits valid or significant growth, and if so determining characteristic transition values such as elbow values in sigmoid or growth-type curves. In one implementation, the systems and methods of the present invention are used for determining the cycle threshold (Ct) value in PCR amplification curves.

In certain aspects, a dataset representing a sigmoid or growth-type curve is processed to determine whether the data exhibits significant or valid growth. In certain aspects, a first or a second degree polynomial curve that fits the data is determined, and a statistical significance value for the curve fit is determined. If the significance value exceeds a significance threshold, the data is considered to not represent significant or valid growth. If the data does not represent significant or valid growth, the data set may be discarded. If the significance value does not exceed the significance threshold, the data is considered to represent significant or valid growth. If the data set is determined to represent valid growth, the data is further processed to determine a transition value in the sigmoid or growth curve, such as the end of the baseline region or the elbow value or Ct value of a PCR amplification curve. In certain aspects, if the data curve representing a growth process is determined to exceed a significance threshold and be judged to represent valid growth, a double sigmoid function with parameters determined by a Levenberg-Marquardt (LM) regression process is used to find an approximation to the curve that fits the dataset. Once the parameters have been determined, the curve can be normalized using one or more of the determined parameters. After normalization, the normalized curve is processed to determine the curvature of the curve at some or all points along the curve, e.g., to produce a dataset or plot representing the curvature vs. the cycle number for a PCR dataset. The cycle number at which the maximum curvature occurs corresponds to the Ct value for a PCR dataset. The curvature and/or the Ct value is then returned and may be displayed or otherwise used for further processing.

According to one aspect of the present invention, a computer implemented method is provided for determining the cycle threshold (Ct) value of a growth curve for a kinetic Polymerase Chain Reaction (PCR) process. The method includes receiving a data set representing a Polymerase Chain reaction (PCR) growth process, the data set including a plurality of data points, each data point having a pair of coordinate values (x,y), where the x-axis represents cycle number and the y-axis fluorescence intensity, and calculating a curve that fits the data set, the curve including a second degree polynomial. The method also includes determining a statistical significance value for the curve, determining whether the significance value exceeds a threshold, and if not, processing the data set further, and if so, indicating that the data set does not have significant growth and/or discarding the data set. Herein, processing the data set further includes determining a cycle threshold (Ct) value of the PCR data set and determining the cycle threshold (Ct) value includes calculating an approximation function that fits the data set by applying a regression process to determine parameters of the function and a curve approximating the data set, wherein the approximation function is a double

$$a + bx + \frac{c}{(1 + \exp^{-d(x-e)})(1 + \exp^{-f(x-g)})}$$

sigmoid function of the form: ; and wherein calculating includes iteratively determining the parameters a, b, c, d, e, f and g of the function by applying a Levenberg-Marquardt (LM) regression process and processing includes determining the curvature at some or all points along the normalized curve; normalizing the curve approximating the data set using the determined parameters to produce a normalized curve; and processing the normalized curve to determine a point of maximum curvature, wherein the point of maximum curvature represents the cycle threshold (Ct) value of the PCR growth curve. In certain aspects, a received dataset includes a dataset that has been processed to remove one or more outliers or spike points. In certain aspects, the statistical significance value is an $R^2$ value, and the threshold is greater than about 0.90. In one aspect, the statistical significance value is an $R^2$ value, and the threshold is about 0.99. In certain embodiments the method further includes normalizing the data set prior to calculating a curve that fits the data set.

According to another aspect of the present invention, a computer-readable medium including code for controlling a processor to determine the cycle threshold (Ct) value of a growth curve for a kinetic Polymerase Chain Reaction (PCR) process is provided. The code includes instructions to receive a data set representing a Polymerase Chain reaction (PCR) growth process, the data set including a plurality of data points, each data point having a pair of coordinate values (x,y), where the x-axis represents cycle number and the y-axis fluorescence intensity, and calculate a curve that fits the data set, the curve including a second degree polynomial. The code also includes instructions to determine a statistical significance value for the curve, determine whether the significance value exceeds a threshold, and if not, process the data set further, and if so, indicate that the data set does not have significant growth and/or discard the data set. Herein, the computer readable medium further includes instructions to determine a cycle threshold (Ct) value of the PCR data set and determining the cycle threshold (Ct) value includes calculating an approximation function that fits the data set by applying a regression process to determine parameters of the function and a curve approximating the data set, wherein the approximation function is a double sigmoid function of the form:

$$a + bx + \frac{c}{(1 + \exp^{-d(x-e)})(1 + \exp^{-f(x-g)})}$$

; and wherein calculating includes iteratively determining the parameters a, b, c, d, e, f and g of the function by applying a Levenberg-Marquardt (LM) regression process and processing includes determining the curvature at some or all points along the normalized curve; normalizing the curve approximating the data set using the determined parameters to produce a normalized curve; and processing the normalized curve to determine a point of maximum curvature, wherein the point of maximum curvature represents the cycle threshold (Ct) value of the PCR growth curve. In certain embodiments, the statistical significance value is an $R^2$ value, and the threshold is greater than about 0.90. In one embodiment, the statistical significance value is an $R^2$ value, and the threshold is about 0.99. The computer readable medium the code further includes instructions to normalize the data set prior to calculating a curve that fits the data set. In yet another implementation the code further includes instructions to output data representing the Ct value. According to yet another aspect of the present invention, a kinetic Polymerase Chain Reaction (PCR) system is provided. The system includes a kinetic PCR analysis module that is adapted to generate a PCR dataset representing a kinetic PCR amplification curve, the dataset including a plurality of data points, each having a pair of coordinate values (x,y), where the x-axis represents cycle number and the y-axis fluorescence intensity, wherein the dataset includes data points in a region of interest which includes a cycle threshold (Ct) value, and an intelligence module adapted to process the PCR data set to determine the cycle threshold (Ct) value of a growth curve for a kinetic Polymerase Chain Reaction (PCR) process. The intelligence module processes the PCR dataset by calculating a curve that fits the PCR data set, the curve including a second degree polynomial, and determining a statistical significance value for the curve. The intelligence module also processes the PCR dataset by determining whether the significance value exceeds a threshold, and if not, processing the PCR data set further, and if so, indicating that the PCR data set does not have significant growth and/or discarding the PCR data set. Herein, processing the data set further includes determining a cycle threshold (Ct) value of the PCR data set and determining the cycle threshold (Ct) value includes calculating an approximation function that fits the data set by applying a regression process to determine parameters of the function and a curve approximating the data set, wherein the approximation function is a double sigmoid function

$$a + bx + \frac{c}{(1 + \exp^{-d(x-e)})(1 + \exp^{-f(x-g)})}$$

of the form: ; and wherein calculating includes iteratively determining the parameters a, b, c, d, e, f and g of the function by applying a Levenberg-Marquardt (LM) regression process and processing includes determining the curvature at some or all points along the normalized curve; normalizing the curve approximating the data set using the determined parameters to produce a normalized curve; and processing the normalized curve to determine a point of maximum curvature, wherein the point of maximum curvature represents the cycle threshold (Ct) value of the PCR growth curve. In certain embodiments, the statistical significance value is an $R^2$ value, and the threshold is greater than about 0.90. In one embodiment, the statistical significance value is an $R^2$ value, and the threshold is about 0.99. The intelligence module is further adapted to normalize the data set prior to calculating a curve that fits the data set. In particular implementations of the PCR system the kinetic PCR analysis module is resident in a kinetic thermocycler device and includes a processor communicably coupled to the analysis module. In certain implementations of the PCR system the intelligence module includes a processor resident in a computer system coupled to the analysis module by one of a network connection or a direct connection.

Reference to the remaining portions of the specification, including the drawings and claims, will realize other features and advantages of the present invention. Further features and advantages of the present invention, as well as the structure and operation of various embodiments of the present invention, are described in detail below with respect to the accompanying drawings. In the drawings, like reference numbers indicate identical or functionally similar elements.

BRIEF DESCRIPTION OF THE DRAWINGS

FIG. 1 illustrates an example of a typical PCR growth curve, plotted as fluorescence intensity vs. cycle number.

FIG. 2 shows a process flow for determining the end of a baseline region of a growth curve, or Ct value of a PCR curve.

FIG. 3 illustrates a detailed process flow for a spike identification and replacement process according to one embodiment of the present invention.

FIG. 4 illustrates a decomposition of the double sigmoid equation including parameters (a)-(g).

FIG. 5 shows the influence of parameter (d) on the curve and the position of (e), the x value of the inflexion point. All curves in FIG. 5 have the same parameter values except for parameter (d).

FIG. 6 shows an example of the three curve shapes for the different parameter sets.

FIG. 7 illustrates a process for determining the value of double sigmoid equation parameters (e) and (g) according to one aspect.

FIG. 8 illustrates a process flow of a Levenberg-Marquardt regression process for an initial set of parameters.

FIG. 9 illustrates a more detailed process flow for determining the elbow value for a PCR process according to one embodiment.

FIG. 10a shows a typical growth curve that was fit to experimental data using a double sigmoid, and FIG. 10b shows a plot of a the curvature of the double sigmoid curve of FIG. 10a.

FIG. 11 shows a circle superimposed in the growth curve in FIG. 10a tangential to the point of maximum curvature.

FIG. 12a shows an example of a data set for a growth curve.

FIG. 12b shows a plot of the data set of FIG. 12a.

FIG. 13 shows a double sigmoid fit to the data set of FIG. 12.

FIG. 14 shows the data set (and double sigmoid fit) of FIG. 12 (FIG. 13) after normalization using the baseline subtraction method of equation (6).

FIG. 15 shows a plot of the curvature vs. cycle number for the normalized data set of FIG. 14.

FIG. 16 shows a superposition of a circle with the maximum radius of curvature and the normalized data set of FIG. 14.

FIG. 17 shows an example of a "slow-grower" data set.

FIG. 18 shows the data set of FIG. 17 and a double sigmoid fit after normalization using the baseline subtraction method of equation (6).

FIG. 19 shows a plot of the curvature vs. cycle number for the normalized data set of FIG. 18.

FIG. 20 shows a plot of a set of PCR growth curves, including replicate runs and negative samples.

FIG. 21 shows a real-time PCR data signal that does not contain a target, and which has a baseline intercept, slope and an AFI value with acceptable ranges.

FIG. 22 shows a real-time PCR data signal having the same (maximum) radius of curvature as the signal in FIG. 21.

FIG. 23 shows a real-time PCR data signal having a low (maximum) radius of curvature.

FIG. 24 shows a general block diagram explaining the relation between the software and hardware resources.

DETAILED DESCRIPTION OF THE INVENTION

The present invention provides systems and methods for determining whether data representing a sigmoid or growth-type curve exhibits significant growth. A second degree polynomial curve that fits the data is determined, and a statistical significance value for the curve fit is determined. If the significance value exceeds a significance threshold, the data is considered to not represent significant or valid growth. If the data does not represent significant or valid growth, the data set may be discarded. If the significance value does not exceed the significance threshold, the data is considered to represent significant or valid growth. If the data set is determined to represent valid growth, the data is further processed to determine a transition value in the sigmoid or growth curve, such as the end of the baseline region or the elbow value or Ct value of a PCR amplification curve. A double sigmoid function with parameters determined by a Levenberg-Marquardt (LM) regression process is used to find an approximation to the curve. Once the parameters have been determined, the curve is normalized using one or more of the determined parameters. After normalization, the normalized curve is processed to determine the curvature of the curve at some or all points along the curve, e.g., to produce a dataset or plot representing the curvature vs. the cycle number. The cycle number at which the maximum curvature occurs corresponds to the Ct value. The Ct value is then returned and may be displayed or otherwise used for further processing.

The method may be implemented by using conventional personal computer systems including, but not limited to, an input device to input a data set, such as a keyboard, mouse, and the like; a display device to represent a specific point of interest in a region of a curve, such as a monitor; a processing device necessary to carry out each step in the method, such as a CPU; a network interface such as a modem, a data storage device to store the data set, a computer code running on the processor and the like. Furthermore, the method may also be implemented in a PCR device.

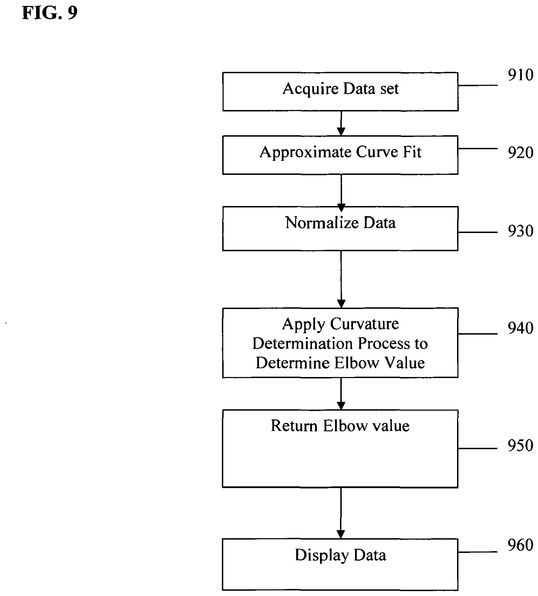

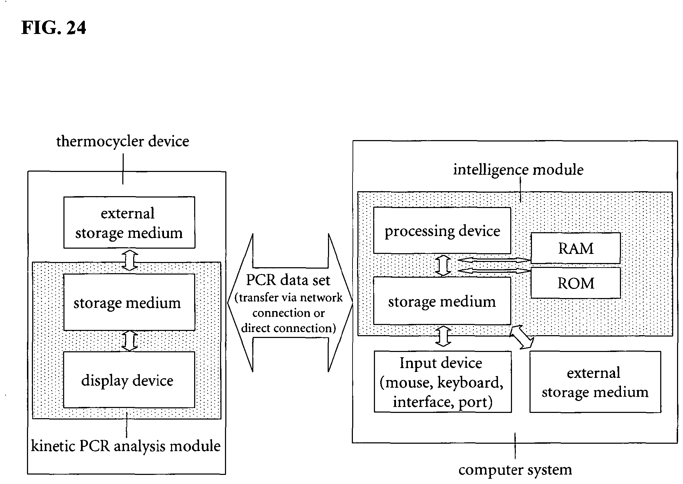

Figure 24 shows a general block diagram explaining the relation between the software and hardware resources. The system may comprise a kinetic PCR analysis module which is located in a thermocycler device and an intelligence module which is part of the computer system. Herein, the data sets (PCR data sets) are transferred from the analysis module to the intelligence module or vice versa via a network connection or a direct connection. The data sets are processed according to the method as displayed in Fig. 2 or 9 by computer code running on the processor and being stored on the storage device of the intelligence module and after processing transferred back to the storage device of the analysis module, where the modified data may be displayed on a displaying device.

Ct Determination for PCR Data with Valid Growth

One example of a growth or amplification curve 10 in the context of a PCR process is shown in FIG. 1. As shown, the curve 10 includes a lag phase region 15, and an exponential phase region 25. Lag phase region 15 is commonly referred to as the baseline or baseline region. Such a curve 10 includes a transitionary region of interest 20 linking the lag phase and the exponential phase regions. Region 20 is commonly referred to as the elbow or elbow region. The elbow region typically defines an end to the baseline and a transition in the growth or amplification rate of the underlying process. Identifying a specific transition point in region 20 can be useful for analyzing the behavior of the underlying process. In a typical PCR curve, identifying a transition point referred to as the elbow value or cycle threshold (Ct) value is useful for understanding efficiency characteristics of the PCR process.

Other processes that may provide similar sigmoid or growth curves include bacterial processes, enzymatic processes and binding processes. In bacterial growth curves, for example, the transition point of interest has been referred to as the time in lag phase, $\theta$. Other specific processes that produce data curves that may be analyzed according to the present invention include strand displacement amplification (SDA) processes, nucleic acid sequence-based amplification (NASBA) processes and transcription mediated amplification (TMA) processes. Examples of SDA and NASBA processes and data curves can be found in Wang, Sha-Sha, et al., "Homogeneous Real-Time Detection of Single-Nucleotide Polymorphisms by Strand Displacement Amplification on the BD ProbeTec ET System", Clin Chem 2003 49(10): 1599, and Weusten, Jos J.A.M., et al., "Principles of Quantitation of Viral Loads Using Nucleic Acid Sequence-Based Amplification in Combination With Homogeneous Detection Using Molecular Beacons", Nucleic Acids Research, 2002 30(6):26, respectively. Thus, although the remainder of this document will discuss embodiments and aspects of the invention in terms of its applicability to PCR curves, it should be appreciated that the present invention may be applied to data curves related to other processes.

As shown in FIG. 1, data for a typical PCR growth curve can be represented in a two-dimensional coordinate system, for example, with PCR cycle number defining the x-axis and an indicator of accumulated polynucleotide growth defining the y-axis. Typically, as shown in FIG. 1, the indicator of accumulated growth is a fluorescence intensity value as the use of fluorescent markers is perhaps the most widely used labeling scheme. However, it should be understood that other indicators may be used depending on the particular labeling and/or detection scheme used. Examples of other useful indicators of accumulated signal growth include luminescence intensity, chemiluminescence intensity, bioluminescence intensity, phosphorescence intensity, charge transfer, voltage, current, power, energy, temperature, viscosity, light scatter, radioactive intensity, reflectivity, transmittance and absorbance. The definition of cycle can also include time, process cycles, unit operation cycles and reproductive cycles.

General Process Overview

According to the present invention, one embodiment of a process 100 for determining a transitionary value in a single sigmoid curve, such as the elbow value or Ct value of a kinetic PCR amplification curve, can be described briefly with reference to FIG. 2. In step 110, an experimental data set representing the curve is received or otherwise acquired. An example of a plotted PCR data set is shown in FIG. 1, where the y-axis and x-axis represent fluorescence intensity and cycle number, respectively, for a PCR curve. In certain aspects, the data set should include data that is continuous and equally spaced along an axis.

In the case where process 100 is implemented in an intelligence module (e.g., processor executing instructions) resident in a PCR data acquiring device such as a thermocycler, the data set may be provided to the intelligence module in real time as the data is being collected, or it may be stored in a memory unit or buffer and provided to the intelligence module after the experiment has been completed. Similarly, the data set may be provided to a separate system such as a desktop computer system or other computer system, via a network connection (e.g., LAN, VPN, intranet, Internet, etc.) or direct connection (e.g., USB or other direct wired or wireless connection) to the acquiring device, or provided on a portable medium such as a CD, DVD, floppy disk or the like.

In certain aspects, the data set includes data points having a pair of coordinate values (or a 2-dimensional vector). For PCR data, the pair of coordinate values typically represents the cycle number and the fluorescence intensity value. After the data set has been received or acquired in step 110, the data set may be analyzed to determine the end of the baseline region.

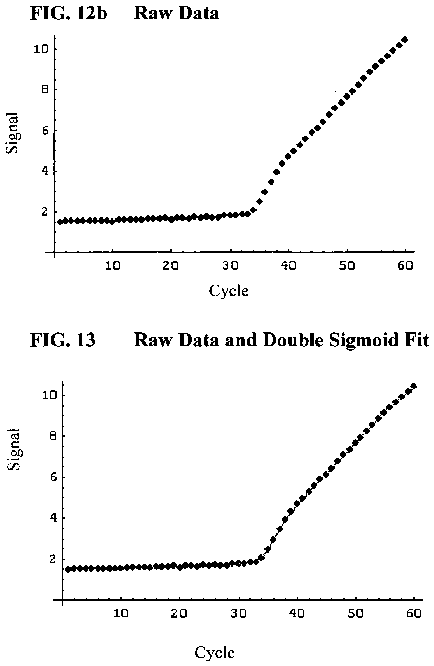

In step 120, an approximation of the curve is calculated. During this step, in one embodiment, a double sigmoid function with parameters determined by a Levenberg-Marquardt (LM) regression process or other regression process is used to find an approximation of a curve representing the data set. The approximation is said to be "robust" as outlier or spike points have a minimal effect on the quality of the curve fit. FIG. 13, which will be discussed below, illustrates an example of a plot of a received data set and a robust approximation of the data set determined by using a Levenberg-Marquardt regression process to determine the parameters of a double sigmoid function according to the present invention.

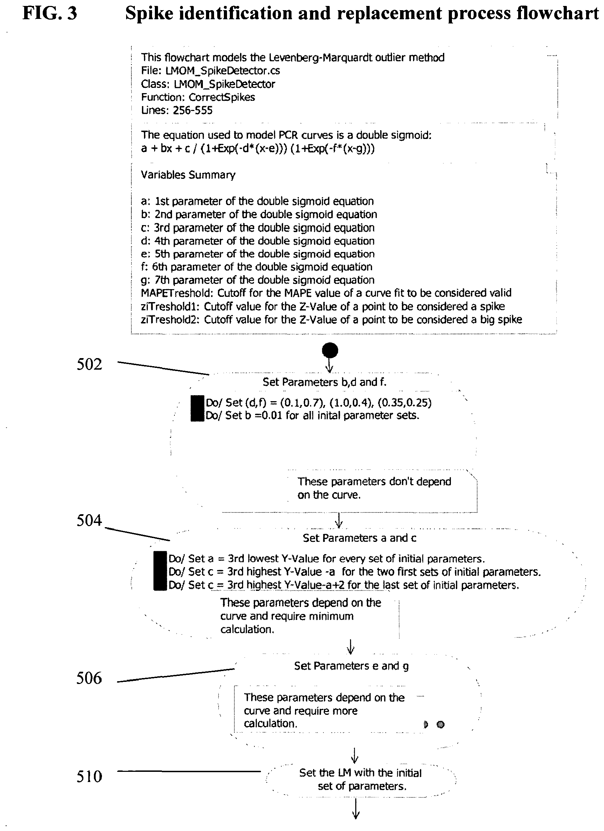

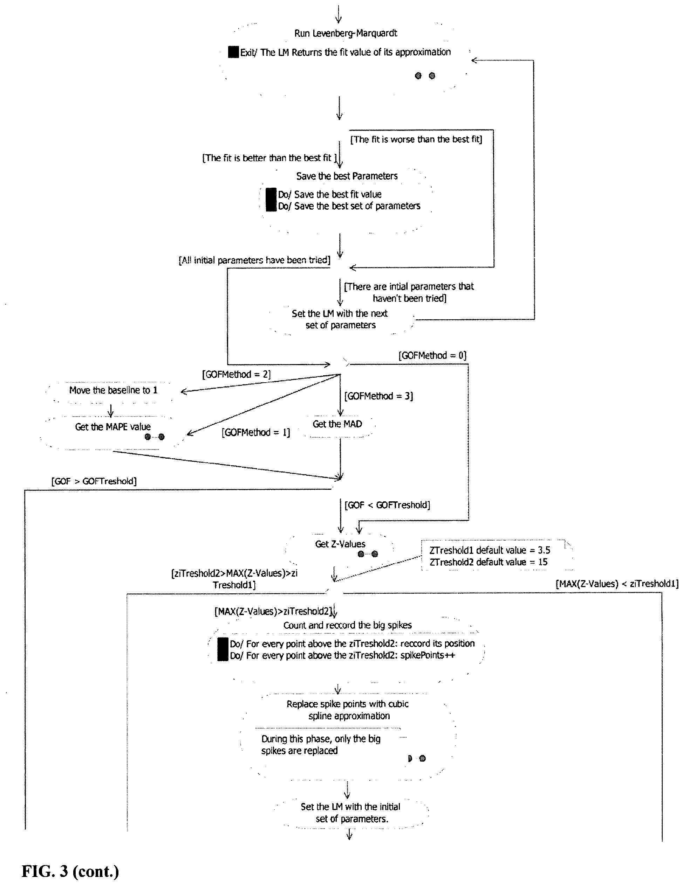

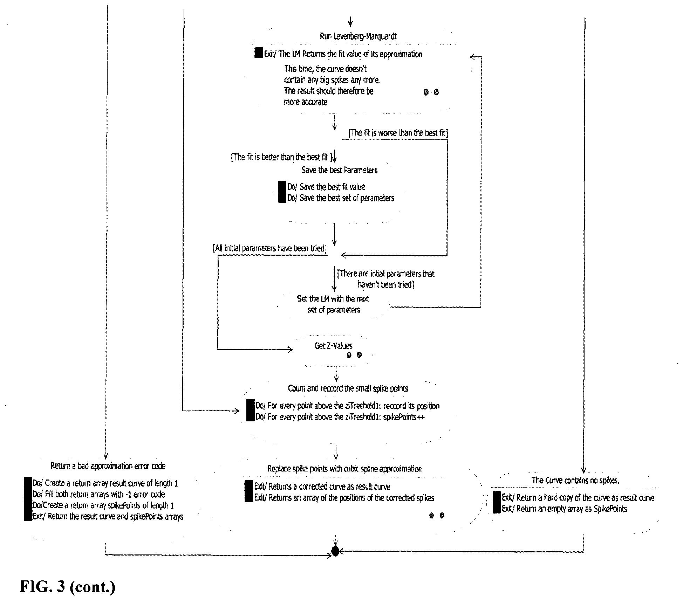

In certain aspects, outlier or spike points in the dataset are removed or replaced prior to processing the data set to determine the end of the baseline region. Spike removal may occur before or after the dataset is acquired in step 110. FIG. 3 illustrates the process flow for identifying and replacing spike points in datasets representing PCR or other growth curves. A more detailed description of a process for determining and removing or replacing spike points can be found in US 2007-0148632.

In step 130, the parameters determined in step 120 are used to normalize the curve, e.g., to remove the baseline slope, as will be described in more detail below. Normalization in this manner allows for determining the Ct value without having to determine or specify the end of the baseline region of the curve or a baseline stop position. In step 140, the normalized curve is then processed to determine the Ct value as will be discussed in more detail below.

LM Regression Process

Steps 502 through 524 of FIG. 3, as will be discussed below, illustrate a process flow for approximating the curve of a dataset and determining the parameters of a fit function (step 120). These parameters can be used in normalizing the curve, e.g., modifying or removing the baseline slope of the data set representing a sigmoid or growth type curve such as a PCR curve according to one embodiment of the present invention (step 130). Where the dataset has been processed to produce a modified dataset with removed or replaced spike points, the modified spikeless dataset may be processed according to steps 502 through 524 to identify the parameters of the fit function.

In one embodiment as shown, a Levenberg-Marquardt (LM) method is used to calculate a robust curve approximation of a data set. The LM method is a non-linear regression process; it is an iterative technique that minimizes the distance between a non-linear function and a data set. The process behaves like a combination of a steepest descent process and a Gauss-Newton process: when the current approximation doesn't fit well it behaves like the steepest descent process (slower but more reliable convergence), but as the current approximation becomes more accurate it will then behave like the Gauss-Newton process (faster but less reliable convergence). The LM regression method is widely used to solve non-linear regression problems.

In general, the LM regression method includes an algorithm that requires various inputs and provides output. In one aspect, the inputs include a data set to be processed, a function that is used to fit the data, and an initial guess for the parameters or variables of the function. The output includes a set of parameters for the function that minimizes the distance between the function and the data set.

According to one embodiment, the fit function is a double sigmoid of the form:

$$f(x) = a + bx + \frac{c}{(1 + \exp^{-d(x-e)})(1 + \exp^{-f(x-g)})}. \qquad (1)$$

The choice of this equation as the fit function is based on its flexibility and its ability to fit the different curve shapes that a typical PCR curve or other growth curve may take. One skilled in the art will appreciate that variations of the above fit function or other fit functions may be used as desired.

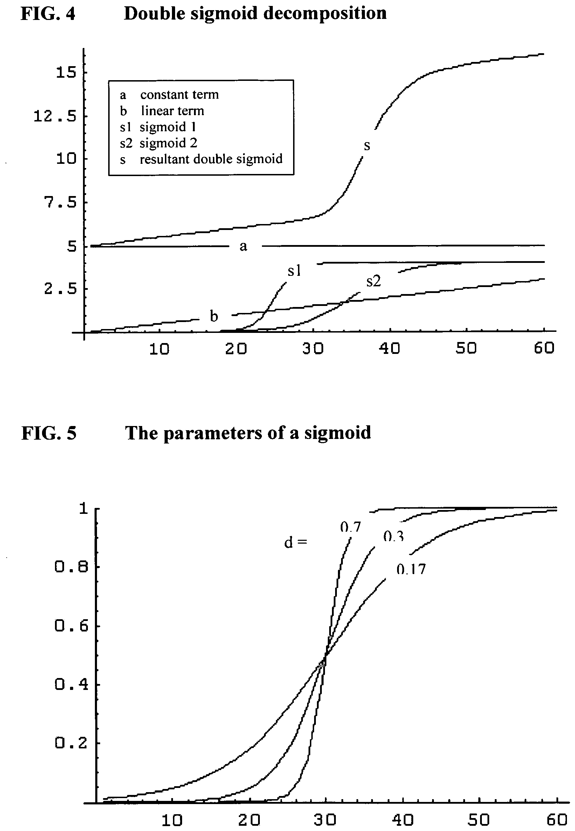

The double sigmoid equation (1) has 7 parameters: a, b, c, d, e, f and g. The equation can be decomposed into a sum of a constant, a slope and a double sigmoid. The double sigmoid itself is the multiplication of two sigmoids. FIG. 4 illustrates a decomposition of the double sigmoid equation (1). The parameters d, e, f and g determine the shape of the two sigmoids. To show their influence on the final curve, consider the single sigmoid:

$$\frac{1}{1 + \exp^{-d(x-e)}}, \qquad (2)$$

where the parameter d determines the "sharpness" of the curve and the parameter e determines the x-value of the inflexion point. FIG. 5 shows the influence of the parameter d on the curve and of the parameter e on the position of the x value of the inflexion point. Table 1, below, describes the influence of the parameters on the double sigmoid curve.

Table 1: Double sigmoid parameters description

| Parameter | Influence on the curve |
|---|---|
| a | Value of y at x = 0 |
| b | baseline and plateau slope |
| c | AFI of the curve |
| d | "sharpness" of the first sigmoid (See Figure 5) |
| e | position of the inflexion point of the first sigmoid (See Figure 5) |
| f | "sharpness" of the second sigmoid |
| g | position of the inflexion point of the second sigmoid |

In one aspect, the "sharpness" parameters d and f of the double sigmoid equation should be constrained in order to prevent the curve from taking unrealistic shapes. Therefore, in one aspect, any iterations where d<-1 or d > 1.1 or where f<-1 or f > 1.1 is considered unsuccessful. In other aspects, different constraints on parameters d and f may be used.

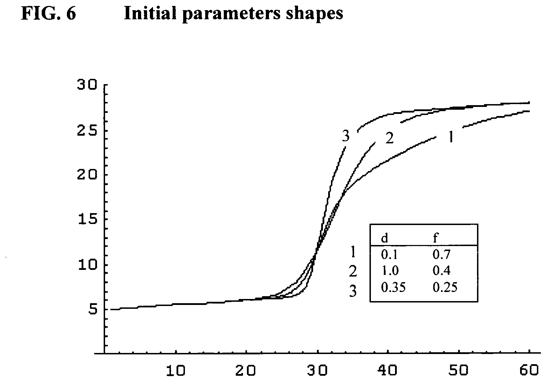

Because the Levenberg-Marquardt algorithm is an iterative algorithm, an initial guess for the parameters of the function to fit is typically needed. The better the initial guess, the better the approximation will be and the less likely it is that the algorithm will converge towards a local minimum. Due to the complexity of the double sigmoid function and the various shapes of PCR curves or other growth curves, one initial guess for every parameter may not be sufficient to prevent the algorithm from sometimes converging towards local minima. Therefore, in one aspect, multiple (e.g., three or more) sets of initial parameters are input and the best result is kept. In one aspect, most of the parameters are held constant across the multiple sets of parameters used; only parameters c, d and f may be different for each of the multiple parameter sets. FIG. 6 shows an example of the three curve shapes for the different parameter sets. The choice of these three sets of parameters is indicative of three possible different shapes of curves representing PCR data. It should be understood that more than three sets of parameters may be processed and the best result kept.

As shown in FIG. 3, the initial input parameters of the LM method are identified in step 510. These parameters may be input by an operator or calculated. According to one aspect, the parameters are determined or set according to steps 502, 504 and 506 as discussed below.

Calculation of initial parameter (a):

The parameter (a) is the height of the baseline; its value is the same for all sets of initial parameters. In one aspect, in step 504 the parameter (a) is assigned the 3rd lowest y-axis value, e.g., fluorescence value, from the data set. This provides for a robust calculation. In other aspects, of course, the parameter (a) may be assigned any other fluorescence value as desired such as the lowest y-axis value, second lowest value, etc.

Calculation of initial parameter (b):

The parameter (b) is the slope of the baseline and plateau. Its value is the same for all sets of initial parameters. In one aspect, in step 502 a static value of 0.01 is assigned to (b) as ideally there shouldn't be any slope. In other aspects, the parameter (b) may be assigned a different value, for example, a value ranging from 0 to about 0.5.

Calculation of initial parameter (c):

The parameter (c) represents the height of the plateau minus the height of the baseline, which is denoted as the absolute fluorescence increase, or AFI. In one aspect, for the first set of parameters, c = AFI + 2, whereas for the last two parameters, c = AFI. This is shown in FIG. 6, where for the last two sets of parameters, c = AFI. For the first set of parameters, c = AFI+2. This change is due to the shape of the curve modeled by the first set of parameters, which doesn't have a plateau.

Calculation of parameters (d) and (f):

The parameters (d) and (f) define the sharpness of the two sigmoids. As there is no way of giving an approximation based on the curve for these parameters, in one aspect three static representative values are used in step 502. It should be understood that other static or non-static values may be used for parameters (d) and/or (f). These pairs model the most common shapes on PCR curves encountered. Table 2, below, shows the values of (d) and (f) for the different sets of parameters as shown in FIG. 6.

Table 2: Values of parameters d and f

| Parameter set number | Value of d | Value of f |
|---|---|---|
| 1 | 0.1 | 0.7 |
| 2 | 1.0 | 0.4 |
| 3 | 0.35 | 0.25 |

Calculation of parameters (e) and (g):

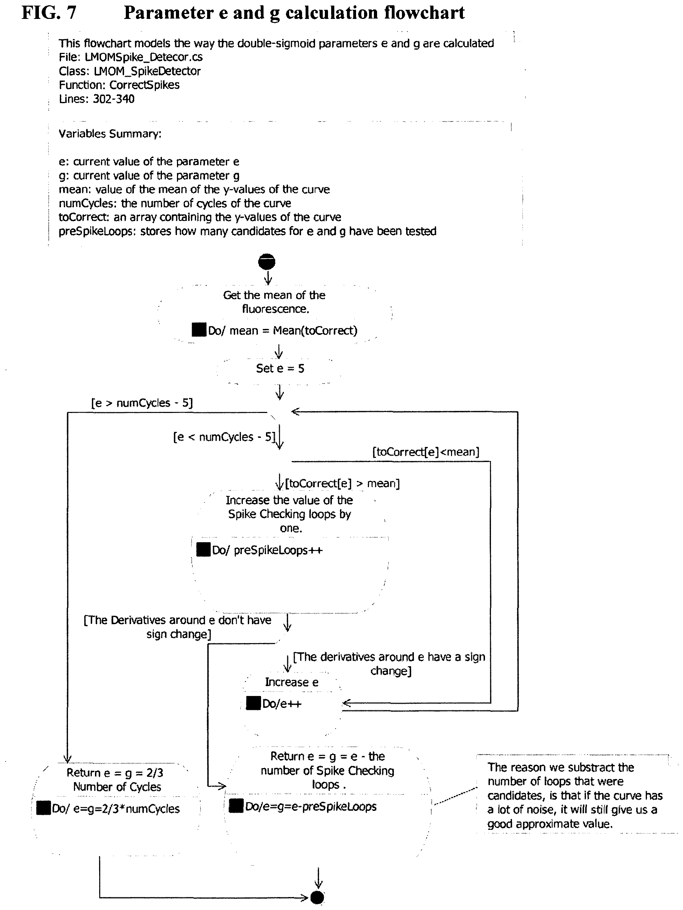

In step 506, the parameters (e) and (g) are determined. The parameters (e) and (g) define the inflexion points of the two sigmoids. In one aspect, they both take the same value across all the initial parameter sets. Parameters (e) and (g) may have the same or different values. To find an approximation, in one aspect, the x-value of the first point above the mean of the intensity, e.g., fluorescence, (which isn't a spike) is used. A process for determining the value of (e) and (g) according to this aspect is shown in FIG. 7 and discussed below. A more detailed description of the process for determining the value of the parameters (e) and (g), and other parameters, according to this aspect can be found in US 2007-0148632.

With reference to FIG. 7, initially, the mean of the curve (e.g., fluorescence intensity) is determined. Next, the first data point above the mean is identified. It is then determined whether:

a. that point does not lie near the beginning, e.g., within the first 5 cycles, of the curve;

b. that point does not lie near the end, e.g., within the 5 last cycles, of the curve; and

c. the derivatives around the point (e.g., in a radius of 2 points around it) do not show any change of sign. If they do, the point is likely to be a spike and should therefore be rejected.

Table 3, below, shows examples of initial parameter values as used in FIG. 6 according to one aspect.

**Table 3: Initial parameters values:**

| Initial parameter set number | 1 | 2 | 3 |
|---|---|---|---|
| Value of a | 3rd lowest fluorescence value | 3rd lowest fluorescence value | 3rd lowest fluorescence value |
| Value of b | 0.01 | 0.01 | 0.01 |
| Value of c | 3rd highest fluorescence value - a + 2 | 3rd highest fluorescence value - a | 3rd highest fluorescence value - a |
| Value of d | 0.1 | 1.0 | 0.35 |
| Value of e | X of the first non-spiky point above the mean of the fluorescence | X of the first non-spiky point above the mean of the fluorescence | X of the first non-spiky point above the mean of the fluorescence |
| Value of f | 0.7 | 0.4 | 0.25 |
| Value of g | X of the first non-spiky point above the mean of the fluorescence | X of the first non-spiky point above the mean of the fluorescence | X of the first non-spiky point above the mean of the fluorescence |

Returning to FIG. 3, once all the parameters are set in step 510, a LM process 520 is executed using the input data set, function and parameters. Traditionally, the Levenberg-Marquardt method is used to solve non-linear least-square problems. The traditional LM method calculates a distance measure defined as the sum of the square of the errors between the curve approximation and the data set. However, when minimizing the sum of the squares, it gives outliers an important weight as their distance is larger than the distance of non-spiky data points, often resulting in inappropriate curves or less desirable curves. Therefore, according to one aspect of the present invention, the distance between the approximation and the data set is computed by minimizing the sum of absolute errors as this does not give as much weight to the outliers. In this aspect, the distance between the approximation and data is given by:

$$\text{distance} = \sum \left| y_{data} - y_{approximation} \right|. \qquad (3)$$

As above, in one aspect, each of the multiple (e.g., three) sets of initial parameters are input and processed and the best result is kept as shown in steps 522 and 524, where the best result is the parameter set that provides the smallest or minimum distance in equation (3). In one aspect, most of the parameters are held constant across the multiple sets of parameters; only c, d and f may be different for each set of parameters. It should be understood that any number of initial parameter sets may be used.

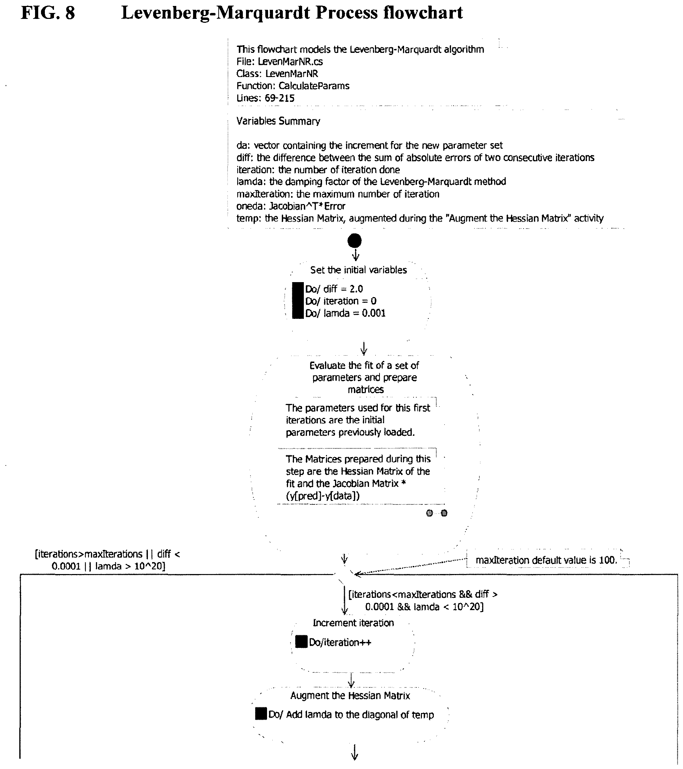

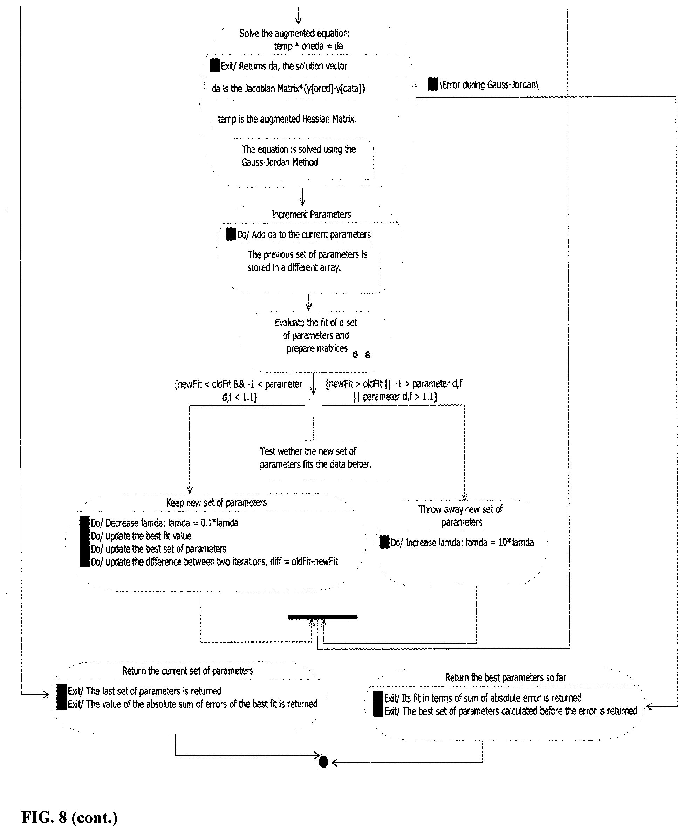

FIG. 8 illustrates a process flow of LM process 520 for a set of parameters according to the present invention. As explained above, the Levenberg-Marquardt method can behave either like a steepest descent process or like a Gauss-Newton process. Its behavior depends on a damping factor $\lambda$. The larger $\lambda$ is, the more the Levenberg-Marquardt algorithm will behave like the steepest descent process. On the other hand, the smaller $\lambda$ is, the more the Levenberg-Marquardt algorithm will behave like the Gauss-Newton process. In one aspect, $\lambda$ is initiated at 0.001. It should be appreciated that $\lambda$ may be initiated at any other value, such as from about 0.000001 to about 1.0.

As stated before, the Levenberg-Marquardt method is an iterative technique. According to one aspect, as shown in FIG. 8 the following is done during each iteration:

1. The Hessian Matrix (H) of the precedent approximation is calculated.

2. The transposed Jacobian Matrix ($J^T$) of the precedent approximation is calculated.

3. The distance vector (d) of the precedent approximation is calculated.

4. The Hessian Matrix diagonal is augmented by the current damping factor $\lambda$:

$$H_{aug} = H\lambda \qquad (4)$$

5. Solve the augmented equation:

$$H_{aug}x = J^T d \qquad (5)$$

6. The solution x of the augmented equation is added to the parameters of the function.

7. Calculate the distance between the new approximation and the curve.

8. If the distance with this new set of parameters is smaller than the distance with the previous set of parameters:

- The iteration is considered successful.

- Keep or store the new set of parameters.

- Decrease the damping factor $\lambda$, e.g., by a factor 10.

If the distance with this new set of parameters is larger than the distance with the previous set of parameters:

- The iteration is considered unsuccessful.

- Throw away the new set of parameters.

- Increase the damping factor $\lambda$, e.g., by a factor of 10.

In one aspect, the LM process of FIG. 8 iterates until one of the following criteria is achieved:

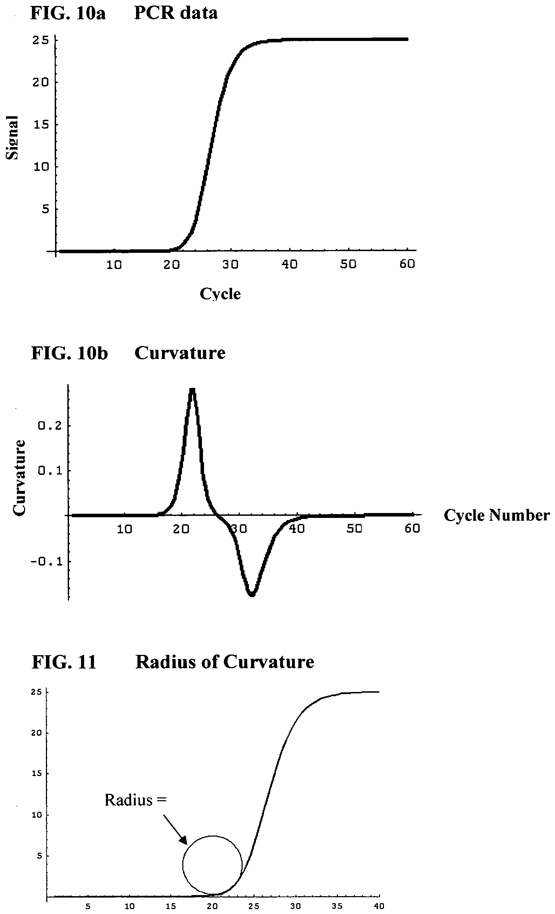

1. It has run for a specified number, N, of iterations. This first criterion prevents the algorithm from iterating indefinitely. For example, in one aspect as shown in FIG. 10, the default iteration value N is 100. 100 iterations should be plenty for the algorithm to converge if it can converge. In general, N can range from fewer than 10 to 100 or more.

2. The difference of the distances between two successful iterations is smaller than a threshold value. e.g., 0.0001. When the difference becomes very small, the desired precision has been achieved and continuing to iterate is pointless as the solution won't become significantly better.

3. The damping factor $\lambda$ exceeds a specified value, e.g., is larger than 1020. When $\lambda$ becomes very large, the algorithm won't converge any better than the current solution, therefore it is pointless to continue iterating. In general, the specified value can be significantly smaller or larger than 1020.

Normalization

After the parameters have been determined, in one embodiment, the curve is normalized (step 130) using one or more of the determined parameters. For example, in one aspect, the curve may be normalized or adjusted to have zero baseline slope by subtracting out the linear growth portion of the curve. Mathematically, this is shown as:

$$\text{dataNew(BLS)} = \text{data-(a+bx)}, \qquad (6)$$

where dataNew(BLS) is the normalized signal after baseline subtraction, e.g., the data set (data) with the linear growth or baseline slope subtracted off or removed. The values of parameters a and b are those values determined by using the LM equation to regress the curve, and x is the cycle number. Thus, for every data value along the x-axis, the constant a and the slope b times the x value is subtracted from the data to produce a data curve with a zero baseline slope. In certain aspects, spike points are removed from the dataset prior to applying the LM regression process to the dataset to determine normalization parameters.

In another aspect, the curve may be normalized or adjusted to have zero slope according to the following equation:

$$\text{dataNew(BLSD)} = \text{(data-(a+bx))/a}, \qquad (7a)$$

where dataNew(BLSD) is the normalized signal after baseline subtraction with division, e.g., the data set (data) with the linear growth or baseline slope subtracted off or removed and the result divided by a. The value of parameters a and b are those values determined by using the LM equation to regress the curve, and x is the cycle number. Thus, for every data value along the x-axis, the constant a and the slope b times the x value is subtracted from the data and the result divided by the value of parameter a to produce a data curve with a zero baseline slope. In one aspect, equation (7a) is valid for parameter "a" $\geq$ 1; in the case where parameter "a" < 1, then the following equation is used:

$$\text{dataNew(BLSD)} = \text{data-(a+bx)}. \qquad (7b)$$

In certain aspects, spike points are removed from the dataset prior to applying the LM regression process to the dataset to determine normalization parameters.
In yet another aspect, the curve may be normalized or adjusted according to following equation:

$$\text{dataNew(BLD)} = \text{data/a}, \qquad (8a)$$

where dataNew(BLD) is the normalized signal after baseline division, e.g., the data set (data) divided by parameter a. The values are the parameters a and b are those values determined by using the LM equation to regress to curve, and x is the cycle number. In one aspect, equation (8a) is valid for parameter "a" $\geq$ 1; in the case where parameter "a" < 1, then the following equation is used:

$$dataNew(BLD) = data + (1\text{-}a). \tag{8b}$$

In certain aspects, spike points are removed from the dataset prior to applying the LM regression process to the dataset to determine normalization parameters.

In yet another aspect, the curve may be normalized or adjusted according to following equation:

$$dataNew(PGT) = (data\text{-}(a+bx))/c, \tag{9a}$$

where dataNew(PGT) is the normalized signal after baseline subtraction with division, e.g., the data set (data) with the linear growth or baseline slope subtracted off or removed and the result divided by c. The value of parameters a, b and c are those values determined by using the LM equation to regress the curve, and x is the cycle number. Thus, for every data value along the x-axis, the constant a and the slope b times the x value is subtracted from the data and the result divided by the value of parameter c to produce a data curve with a zero baseline slope. In one aspect, equation (9a) is valid for parameter "c" $\geq$ 1; in the case where parameter "c" < 1 and "c" $\geq$ 0, then the following equation is used:

$$dataNew(PGT) = data\text{-}(a+bx). \tag{9b}$$

In certain aspects, spike points are removed from the dataset prior to applying the LM regression process to the dataset to determine normalization parameters.

One skilled in the art will appreciate that other normalization equations may be used to normalized and/or modify the baseline using the parameters as determined by the Levenberg-Marquardt regression process.

**Curvature Determination**

After the curve has been normalized using one of equations (6), (7), (8) or (9), or other normalization equation, the Ct value can be determined. In one embodiment, a curvature determination process or method is applied to the normalized curve as will be described with reference to FIG. 9, which shows a process flow for determining the elbow value or Ct value in a kinetic PCR curve. In step 910, the data set is acquired. In the case where the determination process is implemented in an intelligence module (*e.g.,* processor executing instructions) resident in a PCR data acquiring device such as a thermocycler, the data set may be provided to the intelligence module in real time as the data is being collected, or it may be stored in a memory unit or buffer and provided to the module after the experiment has been completed. Similarly, the data set may be provided to a separate system such as a desktop computer system via a network connection (*e.g.,* LAN, VPN, intranet, Internet, *etc.*) or direct connection (*e.g.,* USB or other direct wired or wireless connection) to the acquiring device, or provided on a portable medium such as a CD, DVD, floppy disk or the like.

After a data set has been received or acquired, in step 920 an approximation to the curve is determined. During this step, a double sigmoid function with parameters determined by a Levenberg Marquardt regression process is used to find an approximation of a curve representing the dataset. Additionally, spike points may be removed from the dataset prior to step 920 as described with reference to FIG. 3. For example, the dataset acquired in step 910 can be a dataset with spikes already removed. In step 930, the curve is normalized. In certain aspects, the curve is normalized using one of equations (6), (7), (8) or (9) above. For example, the baseline may be set to zero slope using the parameters of the double sigmoid equation as determined in step with 920 to subtract off the baseline slope as per equation (6) above. In step 940, a process is applied to the normalized curve to determine the curvature at points along the normalized curve. A plot of the curvature vs. cycle number may be returned and/or displayed. The point of maximum curvature corresponds to the elbow or Ct value. In step 950, the result is returned, for example to the system that performed the analysis, or to a separate system that requested the analysis. In step 960, Ct value is displayed. Additional data such as the entire data set or the curve approximation may also be displayed. Graphical displays may be rendered with a display device, such as a monitor screen or printer, coupled with the system that performed the analysis of FIG. 9, or data may be provided to a separate system for rendering on a display device.

To obtain the Ct value for this curve, the maximum curvature is determined. In one aspect, the curvature is determined for some or all points on the normalized curve. A plot of the curvature vs. cycle number may be displayed. The curvature of a curve is given by the equation, below:

$$kappa(x) = \frac{\left(\dfrac{d^2 y}{dx^2}\right)}{\left[1+\left(\dfrac{dy}{dx}\right)^2\right]^{3/2}} \cdot \qquad (10)$$

Consider a circle of radius a, given by the equation below:

$$y(x) = \sqrt{a^2 - x^2} \qquad (11)$$

The curvature of equation (11) is kappa(x) = -(1/a). Thus, the radius of curvature is equal to the negative inverse of the curvature. Since the radius of a circle is constant, its curvature is given by -(1/a). Now consider FIG. 10b, which is a plot of the curvature of the fit of the PCR data set of FIG. 10a. The Ct value can be considered to occur at the position of maximum curvature, which occurs at cycle number Ct = 21.84. This Ct value compares favorably to the PCR growth curve shown in FIG. 10a.

The radius of curvature at the maximum curvature (corresponding to a Ct value of 21.84) is: radius = 1/0.2818 = 3.55 cycles. A circle of this radius superimposed in the PCR growth curve in FIG. 10a is shown in FIG. 11. As FIG. 11 illustrates, a circle of radius corresponding to the maximum curvature represents the largest circle that can be superimposed at the start of the growth region of the PCR curve while remaining tangent to the curve. Curves with a small (maximum) radius of curvature may have steep growth curves while curves with a large (maximum) radius of curvature may have shallow growth curves. If the radius of curvature is extremely large, this may be indicative of curves with no apparent signal, e.g., insignificant growth or non-valid growth. In one embodiment, however, as will be discussed below in more detail, a growth validity test is provided to determine whether the dataset exhibits significant or valid growth. If the data set is found to have statistically significant growth, the curvature analysis algorithm can be applied to determine the Ct value. If not, the dataset may be discarded and/or an indication of invalid growth may be returned.

The first and second derivatives of the double sigmoid of equation (1) that are needed in calculating the curvature are shown below.

**First Derivative**

$$\frac{dy}{dx} = b + \frac{ce^{-f(x-g)}f}{\left(1+e^{-d(x-e)}\right)\left(1+e^{-f(x-g)}\right)^2} + \frac{cde^{-d(x-e)}}{\left(1+e^{-d(x-e)}\right)^2 \left(1+e^{-f(x-g)}\right)} \qquad (12)$$

Equation (13): Second Derivative

$$\frac{d^2 y}{dx^2} = \frac{c\left(\dfrac{2d^2 e^{-2d(x-e)}}{\left(1+e^{-d(x-e)}\right)^3} - \dfrac{d^2 e^{-d(x-e)}}{\left(1+e^{-d(x-e)}\right)^2}\right)}{1+e^{-f(x-g)}} + \frac{2cde^{-d(x-e)-f(x-g)}f}{\left(1+e^{-d(x-e)}\right)^2 \left(1+e^{-f(x-g)}\right)^2} + \frac{c\left(\dfrac{2e^{-2f(x-g)}f^2}{\left(1+e^{-f(x-g)}\right)^3} - \dfrac{e^{-f(x-g)}f^2}{\left(1+e^{-f(x-g)}\right)^2}\right)}{1+e^{-d(x-e)}}$$

**Examples**

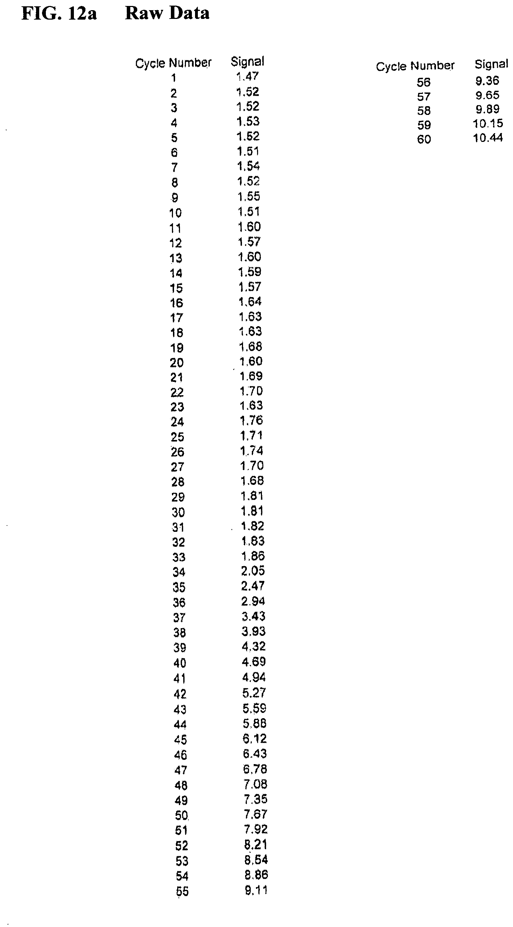

FIG. 12a shows an example of raw data for a growth curve. Applying the double sigmoid/LM method to the raw data plot shown in FIG. 12b gives values of the seven parameters in equation (1) as shown in Table 4 below:

Table 4

| a | 1.4707 |
|---|--------|

(continued)

| | |
|---|---|
| b | 0.0093 |
| c | 10.9421 |
| d | 0.7858 |
| e | 35.9009 |
| f | 0.1081 |
| g | 49.1868 |

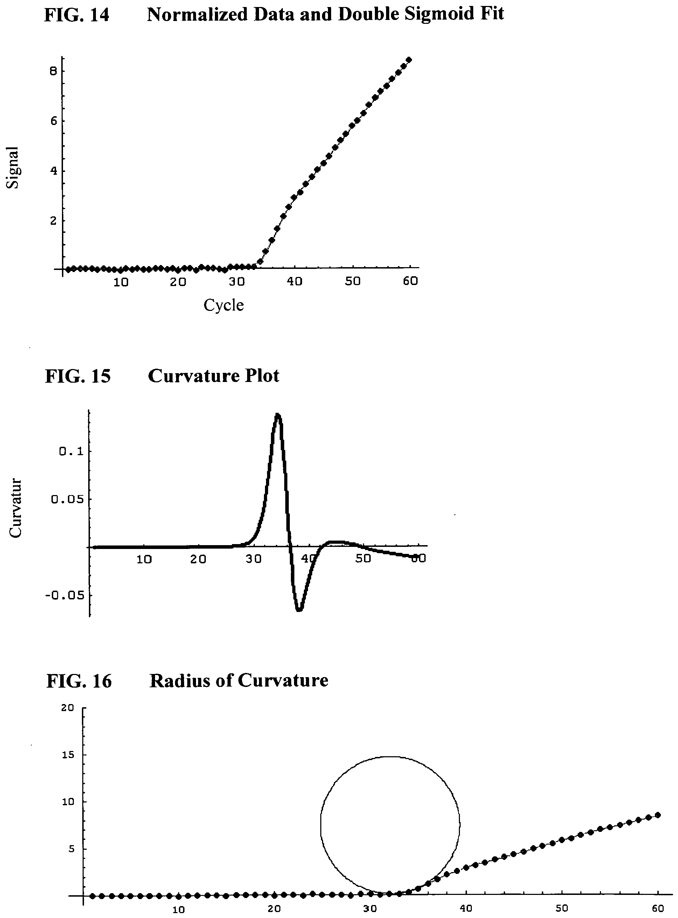

The double sigmoid fit to the data shown in FIG. 12 is shown in FIG. 13, indicating a very accurate assessment of the data points. These data were then normalized according to equation (6) (baseline subtraction) to yield the graph shown in FIG. 14. The solid line shown in FIG. 14 is the double sigmoid/LM application of equation (1) to the data set, which has been normalized according to equation (6). FIG. 15 shows a plot of the curvature vs. cycle number for the normalized curve of FIG. 14. The maximum in the curvature occurs at cycle number 34.42 at a curvature of 0.1378. Thus, $Ct = 34.42$ based on the cycle number at maximum curvature, and the radius of curvature = $1/0.1378 = 7.25$. A superposition of a circle with this radius of curvature and the normalized data set is shown in FIG. 16.

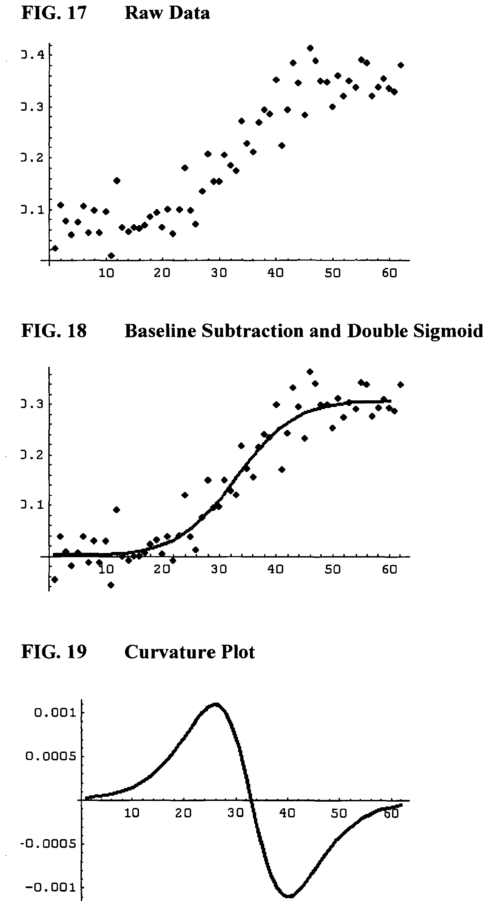

An example of a "slow-grower" data set is shown in FIG. 17. A double sigmoid fit to this data set and normalization using baseline subtraction, equation (6), gives the fit result shown in FIG. 18. The corresponding curvature plot is shown in FIG. 19. The maximum curvature occurs at cycle number 25.90, with a curvature = 0.00109274, corresponding to a radius of curvature = 915. This large radius of curvature communicates that this might be a slow grower data set.

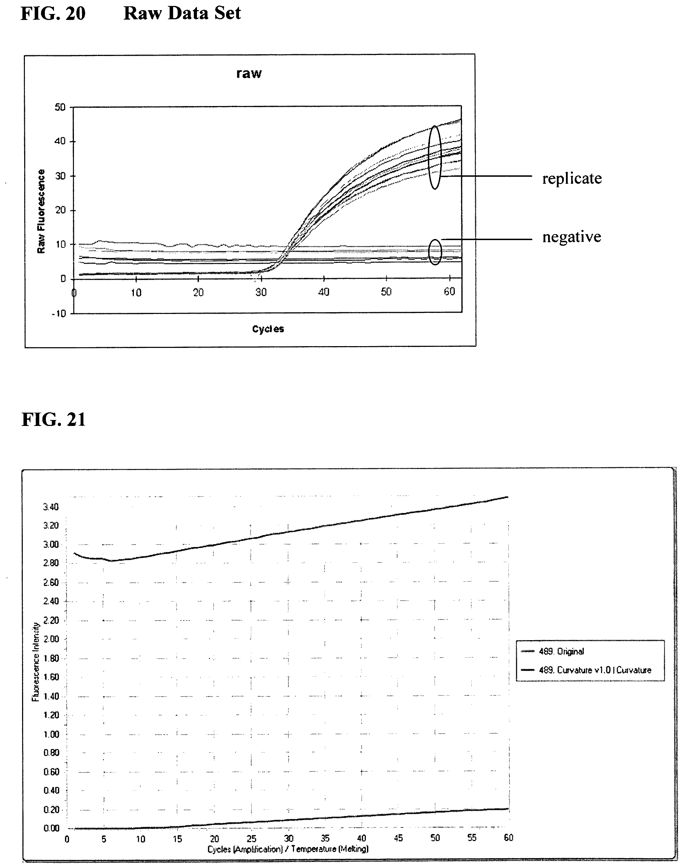

As another example, consider the set of PCR growth curves shown in FIG. 20. A comparison of the Ct values obtained using an existing method ("Threshold") vs. using the curvature method following baseline subtraction with division (BLSD - equation (7)) is shown in Table 5 below.

### Table 5: Ct Values

| Threshold | Curvature Ct BLSD | ROC BLSD |
|:---:|:---:|:---:|
| -3.0 | -3.0 | Infinite |
| -1.0 | 21.3 | 2420.0 |
| -1.0 | -3.0 | Infinite |
| -1.0 | 16.6 | 947.5 |
| -1.0 | 31.6 | 1870.2 |
| -1.0 | 10.3 | 1438.8 |
| -1.0 | 28.1 | 6229.8 |
| -1.0 | 19.8 | 3216.3 |
| 29.1 | 31.7 | 6.0 |
| 29.3 | 31.6 | 5.9 |
| 29.4 | 32.0 | 6.5 |
| 29.5 | 31.7 | 6.4 |
| 29.6 | 31.6 | 5.9 |
| 29.6 | 32.1 | 5.9 |
| 29.9 | 32.0 | 6.1 |
| 30.1 | 31.8 | 6.2 |
| 30.1 | 32.0 | 6.1 |
| 30.2 | 31.9 | 6.1 |
| 30.2 | 31.8 | 6.0 |
| 30.2 | 31.8 | 5.7 |
| 30.3 | 31.7 | 5.9 |
| 30.4 | 31.9 | 6.1 |
| 30.6 | 32.1 | 5.9 |
| 30.6 | 32.2 | 6.2 |
| | | ← Cv |
| **1.56%** | **0.56%** | |

Table 5 indicates that the Curvature method of calculating Ct values (in this case after normalization with BLSD) gives a smaller Cv (coefficient of variation) than the existing Threshold method. In addition, the radius of curvature (ROC) calculated with the curvature method provides a simple method of suggesting whether a curve may be a linear curve or a real growth curve.

Growth Validity Test

In order for Curvature to exist, the PCR signal must be able to be represented by a polynomial of high order (typically a power of 7 or higher as above). If instead, the signal can be represented by a second order polynomial of the form

$$p = a + bx + cx^2 \qquad (14)$$

with an excellent statistical fit (e.g., $R^2 \geq 0.90$), then the curvature for such a signal is determined, in one aspect, as follows:

(1) Perform baseline subtraction on Equation (14), resulting in Equation (15):

$$p = cx^2 \qquad (15)$$

(2) The curvature for Equation (15) is then given as Equation (16):

$$\mathrm{kappa}(x) = \frac{2c}{\left(1 + 4c^2 x^2\right)^{3/2}} \qquad\qquad (16)$$

(3) This Curvature function, Equation (16), has its maximum value at x = 0, therefore implying that there is no defined elbow value for a PCR signal that has an excellent curve fit to a quadratic function. Thus, in one embodiment, if a data set fits a first or second degree polynomial to within a statistically significant margin, the data set is determined to lack significant growth.

A data set for a growth process, is processed to determine whether the data exhibits significant growth. Initially, a second order polynomial curve that fits the data set is calculated (e.g., using equation (14)) and then a statistical significance value is determined for the curve fit. In certain embodiments, the statistical significance is an $R^2$ value. If the statistical significance value does not exceed a threshold value, the data set is judged to exhibit statistically significant or valid growth and the data set is processed further, for example to determine a Ct value. In one embodiment, the $R^2$ threshold is about 0.90; if $R^2$ exceeds 0.90, the data set is judged to be non-valid, e.g., lack significant growth. In another embodiment, the $R^2$ threshold is 0.99. It should be appreciated that the $R^2$ threshold may be set at a value between about 0.90 and 0.99, or that the threshold may be greater than 0.99, or even lower than 0.90. If the statistical significance value does exceed the threshold, the data set is judged to exhibit insignificant, or non-valid, growth. A message indicating that the data set does not have significant growth may be returned and/or the data set may be discarded.

### Examples

FIG. 21 shows a real-time PCR data signal that does not contain a target, and which has a baseline intercept, slope and an AFI value within acceptable ranges. The curvature algorithm of equations (10), (12), and (13) indicates that the Ct value is 12.94 and that the (maximum) radius of curvature (ROC) is 481. When the growth validity test is applied, the data is determined to have insufficient growth or insufficient curvature, meaning that the signal fits a second order quadratic function with a statistical significance value exceeding the threshold, e.g., $R^2 > 0.90$.

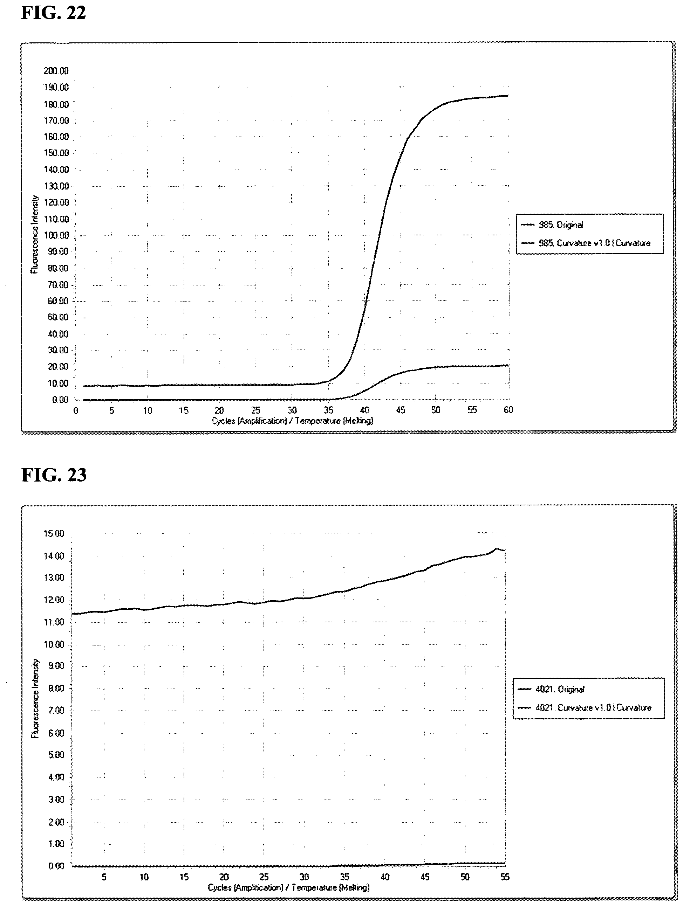

FIG. 22 shows another real-time PCR data signal that also has an ROC of 481; in this case, the $R^2$ value was much less than the threshold, e.g., 0.99, so the process continued to calculate the Ct value. The curvature algorithm of equations (10), (12), and (13) correctly indicates that the maximum radius of curvature, and thus the Ct value, occurs at cycle 38.7. Comparing Figure 21 with FIG. 22, it is apparent that knowledge of the ROC values alone is insufficient to identify whether a curve exhibits valid growth. Here both signals have the same maximum ROC, yet one has valid growth and the other does not.

FIG. 23 shows another real-time PCR signal. Applying the ROC algorithm to determine the Ct value gives a Ct value at cycle 30.3 with a (maximum) ROC of 71. Applying the growth validity test indicates that there is insignificant, or non-valid, growth. Thus, at this much lower (maximum) ROC, the signal is invalid, showing that a low (maximum) ROC in and of itself is insufficient to declare a curve as invalid.

It should be appreciated that the growth validity test and Ct determination processes, including the curve fitting and curvature determination processes, may be implemented in computer code running on a processor of a computer system. The code includes instructions for controlling a processor to implement various aspects and steps of the growth validity Ct determination processes. The code is typically stored on a hard disk, RAM or portable medium such as a CD, DVD, etc. Similarly, the processes may be implemented in a PCR device such as a thermocycler including a processor executing instructions stored in a memory unit coupled to the processor. Code including such instructions may be downloaded to the PCR device memory unit over a network connection or direct connection to a code source or using a portable medium as is well known.

One skilled in the art should appreciate that the elbow determination processes of the present invention can be coded using a variety of programming languages such as C, C++, C#, Fortran, VisualBasic, etc., as well as applications such as Mathematica which provide pre-packaged routines, functions and procedures useful for data visualization and analysis. Another example of the latter is MATLAB®.

### Claims

1. A computer-implemented method of determining the cycle threshold (Ct) value of a growth curve for a kinetic Polymerase Chain Reaction (PCR) process, the method comprising:

   - receiving a data set representing a Polymerase Chain reaction (PCR) growth process, the data set including a plurality of data points, each data point having a pair of coordinate values (x,y), where the x-axis represents cycle number and the y-axis fluorescence intensity;
- calculating a curve that fits the data set, said curve including a second degree polynomial;
- determining a statistical significance value for said curve;
- determining whether the significance value exceeds a threshold; and
- if not, processing the data set further; and
- if so, indicating that the data set does not have significant growth and/or discarding the data set,
wherein processing the data set further includes determining a cycle threshold (Ct) value of the PCR data set; and
wherein determining the cycle threshold (Ct) value includes:

- calculating an approximation function that fits the data set by applying a regression process to determine parameters of the function and a curve approximating the data set, wherein the approximation function is a double sigmoid function of the form:

$$a + bx + \frac{c}{(1 + \exp^{-d(x-e)})(1 + \exp^{-f(x-g)})} ;$$

and wherein calculating includes iteratively determining the parameters a, b, c, d, e, f and g of the function by applying a Levenberg-Marquardt (LM) regression process and processing includes determining the curvature at some or all points along the normalized curve;
- normalizing the curve approximating the data set using the determined parameters to produce a normalized curve; and
- processing the normalized curve to determine a point of maximum curvature, wherein the point of maximum curvature represents the cycle threshold (Ct) value of the PCR growth curve.

2. The method of claim 1, wherein the second degree polynomial is of the form:

$$p = a + bx + cx^2 .$$

3. The method of claims 1 or 2, wherein the statistical significance value is an $R^2$ value, and wherein the threshold is about 0.90 or greater.

4. The method of any one of claims 1 to 3, further including normalizing the data set prior to calculating a curve that fits the data set.

5. A computer-readable medium including code for controlling a processor to determine the cycle threshold (Ct) value of a growth curve for a kinetic Polymerase Chain Reaction (PCR) process, the code including instructions to:

- receive a data set representing a Polymerase Chain reaction (PCR) growth process, the data set including a plurality of data points, each data point having a pair of coordinate values (x,y), where the x-axis represents cycle number and the y-axis fluorescence intensity;
- calculate a curve that fits the data set, said curve including a second degree polynomial;
- determine a statistical significance value for said curve;
- determine whether the significance value exceeds a threshold; and
- if not, process the data set further; and
- if so, indicate that the data set does not have significant growth and/or discard the data set,
wherein processing the data set further includes determining a cycle threshold (Ct) value of the PCR data set; and
wherein determining the cycle threshold (Ct) value includes:

- calculating an approximation function that fits the data set by applying a regression process to determine parameters of the function and a curve approximating the data set, wherein the approximation function is a double sigmoid function of the form:

$$a + bx + \frac{c}{(1 + \exp^{-d(x-e)})(1 + \exp^{-f(x-g)})} ;$$

and wherein calculating includes iteratively determining the parameters a, b, c, d, e, f and g of the function by applying a Levenberg-Marquardt (LM) regression process and processing includes determining the curvature at some or all points along the normalized curve;

- normalizing the curve approximating the data set using the determined parameters to produce a normalized curve; and
- processing the normalized curve to determine a point of maximum curvature, wherein the point of maximum curvature represents the cycle threshold (Ct) value of the PCR growth curve.

6. The computer readable medium of claim 5, wherein the second degree polynomial is of the form:

$$p = a + bx + cx^2$$ .

7. The computer readable medium of claims 5 or 6, wherein the statistical significance value is an $R^2$ value, and wherein the threshold is about 0.90 or greater.

8. A kinetic Polymerase Chain Reaction (PCR) system, comprising:

- a kinetic PCR analysis module adapted to generate a PCR data set representing a kinetic PCR amplification curve, said data set including a plurality of data points, each having a pair of coordinate values (x,y), where the x-axis represents cycle number and the y-axis fluorescence intensity; and
- an intelligence module adapted to process the PCR data set to determine the cycle threshold (Ct) value of a growth curve for a kinetic Polymerase Chain Reaction (PCR) process, by:
- calculating a curve that fits the PCR data set, said curve including a second degree polynomial;
- determining a statistical significance value for said curve;
- determining whether the significance value exceeds a threshold; and
- if not, processing the PCR data set further; and
- if so, indicating that the PCR data set does not have significant growth and/or discarding the PCR data set, wherein processing the data set further includes determining a cycle threshold (Ct) value of the PCR data set; and wherein determining the cycle threshold (Ct) value includes:

- calculating an approximation function that fits the data set by applying a regression process to determine parameters of the function and a curve approximating the data set, wherein the approximation function is a double sigmoid function of the form:

$$a + bx + \frac{c}{(1 + \exp^{-d(x-e)})(1 + \exp^{-f(x-g)})}$$ ;

and wherein calculating includes iteratively determining the parameters a, b, c, d, e, f and g of the function by applying a Levenberg-Marquardt (LM) regression process and processing includes determining the curvature at some or all points along the normalized curve;

- normalizing the curve approximating the data set using the determined parameters to produce a normalized curve; and
- processing the normalized curve to determine a point of maximum curvature, wherein the point of maximum curvature represents the cycle threshold (Ct) value of the PCR growth curve.

9. The PCR system of claim 8, wherein the second degree polynomial is of the form:

$$p = a + bx + cx^2$$ .

10. The PCR system of claims 8 or 9, wherein the statistical significance value is an R2 value, and wherein the threshold is about 0.90 or greater.

**Patentansprüche**

1. Computerimplementiertes Verfahren zum Bestimmen des Zyklusschwellen-(Ct)-Wertes einer Wachstumskurve für einen kinetischen Polymerasekettenreaktion-(PCR)-Prozess, wobei das Verfahren Folgendes umfasst:

   - Empfangen eines Datensatzes, der einen Polymerasekettenreaktion-(PCR)-Wachstumsprozess darstellt, wobei der Datensatz eine Vielzahl von Datenpunkten einschließt, wobei jeder Datenpunkt ein Paar von Koordinatenwerten (x,y) aufweist, wobei die x-Achse die Zykluszahl und die y-Achse die Fluoreszenzintensität darstellt,
   - Berechnen einer Kurve, die dem Datensatz entspricht, wobei die Kurve ein Polynom zweiten Grades einschließt;
   - Bestimmen eines statistischen Signifikanzwertes für die Kurve,
   - Bestimmen, ob der Signifikanzwert eine Schwelle überschreitet; und
   - wenn nicht, Weiterverarbeiten des Datensatzes, und
   - wenn ja, Angeben, dass der Datensatz kein signifikantes Wachstum aufweist und/oder Verwerfen des Datensatzes,
   wobei das Weiterverarbeiten des Datensatzes das Bestimmen eines Zyklusschwellen-(Ct)-Wertes des PCR-Datensatzes einschließt; und
   wobei das Bestimmen des Zyklusschwellen-(Ct)-Wertes Folgendes einschließt:

   - Berechnen einer Näherungsfunktion, die dem Datensatz entspricht, durch Anwenden eines Regressionsprozesses zum Bestimmen von Parametern der Funktion und einer Kurve, die sich dem Datensatz annähert, wobei die Näherungsfunktion eine Doppelsigmoidfunktion der folgenden Form ist:

$$a + bx + \frac{c}{(1 + \exp^{-d(x-e)})(1 + \exp^{-f(x-g)})};$$

   und wobei das Berechnen das iterative Bestimmen der Parameter a, b, c, d, e, f und g der Funktion durch Anwenden eines Levenberg-Marquardt-(LM)-Regressionsprozesses einschließt und das Verarbeiten das Bestimmen der Krümmung an einigen oder allen Punkten entlang der normalisierten Kurve einschließt;
   - Normalisieren der Kurve, die sich dem Datensatz annähert, unter Verwendung der bestimmten Parameter zur Erzeugung einer normalisierten Kurve, und
   - Verarbeiten der normalisierten Kurve zum Bestimmen eines Punktes der maximalen Krümmung, wobei der Punkt der maximalen Krümmung den Zyklusschwellen-(Ct)-Wert der PCR-Wachstumskurve darstellt.

2. Verfahren nach Anspruch 1, wobei das Polynom zweiten Grades die folgende Form aufweist:

$$p = a + bx + cx^2.$$

3. Verfahren nach den Ansprüchen 1 oder 2, wobei der statistische Signifikanzwert ein $R^2$-Wert ist und wobei die Schwelle etwa 0,90 oder mehr beträgt.

4. Verfahren nach einem der Ansprüche 1 bis 3, das ferner das Normalisieren des Datensatzes vor dem Berechnen einer Kurve, die dem Datensatz entspricht, einschließt.

5. Computerlesbares Medium, das einen Code zum Steuern eines Prozessors zum Bestimmen des Zyklusschwellen-(Ct)-Wertes einer Wachstumskurve für einen kinetischen Polymerasekettenreaktion-(PCR)-Prozess einschließt, wobei der Code die folgenden Anweisungen einschließt:

   - Empfangen eines Datensatzes, der einen Polymerasekettenreaktion-(PCR)-Wachstumsprozess darstellt, wobei der Datensatz eine Vielzahl von Datenpunkten einschließt, wobei jeder Datenpunkt ein Paar von Koordinatenwerten (x,y) aufweist, wobei die x-Achse die Zykluszahl und die y-Achse die Fluoreszenzintensität darstellt,
   - Berechnen einer Kurve, die dem Datensatz entspricht, wobei die Kurve ein Polynom zweiten Grades einschließt;
   - Bestimmen eines statistischen Signifikanzwertes für die Kurve,
   - Bestimmen, ob der Signifikanzwert eine Schwelle überschreitet; und
   - wenn nicht, Weiterverarbeiten des Datensatzes, und

- wenn ja, Angeben, dass der Datensatz kein signifikantes Wachstum aufweist und/oder Verwerfen des Datensatzes,
wobei das Weiterverarbeiten des Datensatzes das Bestimmen eines Zyklusschwellen-(Ct)-Wertes des PCR-Datensatzes einschließt; und
wobei das Bestimmen des Zyklusschwellen-(Ct)-Wertes Folgendes einschließt:

- Berechnen einer Näherungsfunktion, die dem Datensatz entspricht, durch Anwenden eines Regressionsprozesses zum Bestimmen von Parametern der Funktion und einer Kurve, die sich dem Datensatz annähert, wobei die Näherungsfunktion eine Doppelsigmoidfunktion der folgenden Form ist:

$$a + bx + \frac{c}{(1 + \exp^{-d(x-e)})(1 + \exp^{-f(x-g)})};$$

und wobei das Berechnen das iterative Bestimmen der Parameter a, b, c, d, e, f und g der Funktion durch Anwenden eines Levenberg-Marquardt-(LM)-Regressionsprozesses einschließt und das Verarbeiten das Bestimmen der Krümmung an einigen oder allen Punkten entlang der normalisierten Kurve einschließt;
- Normalisieren der Kurve, die sich dem Datensatz annähert, unter Verwendung der bestimmten Parameter zur Erzeugung einer normalisierten Kurve, und
- Verarbeiten der normalisierten Kurve zum Bestimmen eines Punktes der maximalen Krümmung, wobei der Punkt der maximalen Krümmung den Zyklusschwellen-(Ct)-Wert der PCR-Wachstumskurve darstellt.

6. Computerlesbares Medium nach Anspruch 5, wobei das Polynom zweiten Grades die folgende Form aufweist:

$$p = a + bx + cx^2.$$

7. Computerlesbares Medium nach den Ansprüchen 5 oder 6, wobei der statistische Signifikanzwert ein $R^2$-Wert ist und wobei die Schwelle etwa 0,90 oder mehr beträgt.

8. Kinetisches Polymerasekettenreaktion-(PCR)-System, umfassend:

- ein kinetisches PCR-Analysemodul, das dafür ausgelegt ist, einen PCR-Datensatz zu erzeugen, der eine kinetische PCR-Amplifikationskurve darstellt, wobei der Datensatz eine Vielzahl von Datenpunkten einschließt, die ein Paar von Koordinatenwerten (x,y) aufweisen, wobei die x-Achse die Zykluszahl und die y-Achse die Fluoreszenzintensität darstellt, und
- ein Intelligenzmodul, das dafür ausgelegt ist, den PCR-Datensatz zu verarbeiten, um den Zyklusschwellen-(Ct)-Wert einer Wachstumskurve für einen kinetischen Polymerasekettenreaktion-(PCR)-Prozess zu bestimmen durch:
- Berechnen einer Kurve, die dem PCR-Datensatz entspricht, wobei die Kurve ein Polynom zweiten Grades einschließt;
- Bestimmen eines statistischen Signifikanzwertes für die Kurve,
- Bestimmen, ob der Signifikanzwert eine Schwelle überschreitet; und
- wenn nicht, Weiterverarbeiten des PCR-Datensatzes, und
- wenn ja, Angeben, dass der PCR-Datensatz kein signifikantes Wachstum aufweist und/oder Verwerfen des PCR-Datensatzes,
wobei das Weiterverarbeiten des Datensatzes das Bestimmen eines Zyklusschwellen-(Ct)-Wertes des PCR-Datensatzes einschließt; und
wobei das Bestimmen des Zyklusschwellen-(Ct)-Wertes Folgendes einschließt:

- Berechnen einer Näherungsfunktion, die dem Datensatz entspricht, durch Anwenden eines Regressionsprozesses zum Bestimmen von Parametern der Funktion und einer Kurve, die sich dem Datensatz annähert, wobei die Näherungsfunktion eine Doppelsigmoidfunktion der folgenden Form ist:

$$a + bx + \frac{c}{(1 + \exp^{-d(x-e)})(1 + \exp^{-f(x-g)})};$$

und wobei das Berechnen das iterative Bestimmen der Parameter a, b, c, d, e, f und g der Funktion durch Anwenden eines Levenberg-Marquardt-(LM)-Regressionsprozesses einschließt und das Verarbeiten das Bestimmen der Krümmung an einigen oder allen Punkten entlang der normalisierten Kurve einschließt;
- Normalisieren der Kurve, die sich dem Datensatz annähert, unter Verwendung der bestimmten Parameter zur Erzeugung einer normalisierten Kurve, und
- Verarbeiten der normalisierten Kurve zum Bestimmen eines Punktes der maximalen Krümmung, wobei der Punkt der maximalen Krümmung den Zyklusschwellen-(Ct)-Wert der PCR-Wachstumskurve darstellt.

9. PCR-System nach Anspruch 8, wobei das Polynom zweiten Grades die folgende Form aufweist:

$$p = a + bx + cx^2.$$

10. PCR-System nach den Ansprüchen 8 oder 9, wobei der statistische Signifikanzwert ein R2-Wert ist und wobei die Schwelle etwa 0,90 oder mehr beträgt.


**Revendications**

1. Procédé mis en oeuvre par ordinateur de détermination de la valeur de cycle seuil (Ct) d'une courbe de croissance pour un procédé de réaction en chaîne par polymérase (PCR) cinétique, le procédé comprenant :

- la réception d'un jeu de données représentant un procédé de croissance par réaction en chaîne par polymérase (PCR), ledit jeu de données incluant une pluralité de points de données, chaque point de données ayant une paire de valeurs de coordonnées (x,y), où l'axe des x représente le nombre de cycles et l'axe des y l'intensité de fluorescence ;
- le calcul d'une courbe qui s'ajuste au jeu de données, ladite courbe incluant un polynôme du second degré ;
- la détermination d'une valeur de signification statistique pour ladite courbe ;
- la détermination si la valeur de signification dépasse un seuil ; et
- si non, le traitement ultérieur du jeu de données ; et
- si oui, l'indication que le jeu de données n'a pas de croissance significative et/ou le rejet du jeu de données, dans lequel le traitement ultérieur du jeu de données inclut la détermination d'une valeur de cycle seuil (Ct) du jeu de données de PCR ; et
dans lequel la détermination de la valeur de cycle seuil (Ct) inclut :

- le calcul d'une fonction d'approximation qui s'ajuste au jeu de données par application d'un procédé de régression pour déterminer les paramètres de la fonction et une courbe approximant le jeu de données, dans lequel la fonction d'approximation est une fonction double sigmoïde de la forme :

$$a + bx + \frac{c}{(1 + \exp^{-d(x-e)})(1 + \exp^{-f(x-g)})} ;$$

et dans lequel le calcul inclut la détermination de façon itérative des paramètres a, b, c, d, e, f et g de la fonction par application d'un procédé de régression de Levenberg-Marquardt (LM) et le traitement inclut la détermination de la courbure en certains ou tous les points le long de la courbe normalisée ;
- la normalisation de la courbe approximant le jeu de données en utilisant les paramètres déterminés pour produire une courbe normalisée ; et
- le traitement de la courbe normalisée pour déterminer un point de courbure maximale, dans lequel le point de courbure maximale représente la valeur de cycle seuil (Ct) de la courbe de croissance par PCR.

2. Procédé selon la revendication 1, dans lequel le polynôme du second degré est de la forme :

$$p = a + bx + cx^2.$$

3. Procédé selon les revendications 1 ou 2, dans lequel la valeur de signification statistique est une valeur $R^2$, et dans lequel le seuil est d'environ 0,90 ou plus.

4. Procédé selon l'une quelconque des revendications 1 à 3, incluant en outre la normalisation du jeu de données avant le calcul d'une courbe qui s'ajuste au jeu de données.

5. Support lisible par ordinateur incluant un code pour commander un processeur afin qu'il détermine la valeur de cycle seuil (Ct) d'une courbe de croissance pour un procédé de réaction en chaîne par polymérase (PCR) cinétique, le code incluant des instructions pour :

- recevoir un jeu de données représentant un procédé de croissance par réaction en chaîne par polymérase (PCR), le jeu de données incluant une pluralité de points de données, chaque point de données ayant une paire de valeurs de coordonnées (x,y), où l'axe des x représente le nombre de cycles et l'axe des y l'intensité de fluorescence ;
- calculer une courbe qui s'ajuste au jeu de données, ladite courbe incluant un polynôme du second degré ;
- déterminer une valeur de signification statistique pour ladite courbe ;
- déterminer si la valeur de signification dépasse un seuil ; et
- si non, traiter ultérieurement le jeu de données ; et
- si oui, indiquer que le jeu de données n'a pas de croissance significative et/ou rejeter le jeu de données,
dans lequel le traitement ultérieur du jeu de données inclut la détermination d'une valeur de cycle seuil (Ct) du jeu de données de PCR ; et
dans lequel la détermination de la valeur de cycle seuil (Ct) inclut :

- le calcul d'une fonction d'approximation qui s'ajuste au jeu de données par application d'un procédé de régression pour déterminer les paramètres de la fonction et une courbe approximant le jeu de données, dans lequel la fonction d'approximation est une fonction double sigmoïde de la forme :

$$a + bx + \frac{c}{(1 + \exp^{-d(x-e)})(1 + \exp^{-f(x-g)})} \; ;$$

et dans lequel le calcul inclut la détermination de façon itérative des paramètres a, b, c, d, e, f et g de la fonction par application d'un procédé de régression de Levenberg-Marquardt (LM) et le traitement inclut la détermination de la courbure en certains ou tous les points le long de la courbe normalisée ;
- la normalisation de la courbe approximant le jeu de données en utilisant les paramètres déterminés pour produire une courbe normalisée ; et
- le traitement de la courbe normalisée pour déterminer un point de courbure maximale, dans lequel le point de courbure maximale représente la valeur de cycle seuil (Ct) de la courbe de croissance par PCR.

6. Support lisible par ordinateur selon la revendication 5, dans lequel le polynôme du second degré est de la forme :

$$p = a + bx + cx^2.$$

7. Support lisible par ordinateur selon les revendications 5 ou 6, dans lequel la valeur de signification statistique est une valeur $R^2$, et dans lequel le seuil est d'environ 0,90 ou plus.

8. Système de réaction en chaîne par polymérase (PCR) cinétique, comprenant :

- un module d'analyse par PCR cinétique adapté pour générer un jeu de données de PCR représentant une courbe d'amplification par PCR cinétique, ledit jeu de données incluant une pluralité de points de données, chacun ayant une paire de valeurs de coordonnées (x,y), où l'axe des x représente le nombre de cycles et l'axe des y l'intensité de fluorescence ; et
- un module d'intelligence adapté pour traiter le jeu de données de PCR afin de déterminer la valeur de cycle seuil (Ct) d'une courbe de croissance pour un procédé de réaction en chaîne par polymérase (PCR), par :
- le calcul d'une courbe qui s'ajuste au jeu de données de PCR, ladite courbe incluant un polynôme du second degré ;
- la détermination d'une valeur de signification statistique pour ladite courbe ;
- la détermination si la valeur de signification dépasse un seuil ; et
- si non, le traitement ultérieur du jeu de données de PCR ; et
- si oui, l'indication que le jeu de données de PCR n'a pas de croissance significative et/ou le rejet du jeu de données de PCR,

dans lequel le traitement ultérieur du jeu de données inclut la détermination d'une valeur de cycle seuil (Ct) du jeu de données de PCR ; et

dans lequel la détermination de la valeur de cycle seuil (Ct) inclut :

- le calcul d'une fonction d'approximation qui s'ajuste au jeu de données par application d'un procédé de régression pour déterminer les paramètres de la fonction et une courbe approximant le jeu de données, dans lequel la fonction d'approximation est une fonction double sigmoïde de la forme :

$$a + bx + \frac{c}{(1 + \exp^{-d(x-e)})(1 + \exp^{-f(x-g)})} \; ;$$

et dans lequel le calcul inclut la détermination de façon itérative des paramètres a, b, c, d, e, f et g de la fonction par application d'un procédé de régression de Levenberg-Marquardt (LM) et le traitement inclut la détermination de la courbure en certains ou tous les points le long de la courbe normalisée ;

- la normalisation de la courbe approximant le jeu de données en utilisant les paramètres déterminés pour produire une courbe normalisée ; et

- le traitement de la courbe normalisée pour déterminer un point de courbure maximale, dans lequel le point de courbure maximale représente la valeur de cycle seuil (Ct) de la courbe de croissance par PCR.

**9.** Système de PCR selon la revendication 8, dans lequel le polynôme du second degré est de la forme :

$$p = a + bx + cx^2.$$

**10.** Système de PCR selon les revendications 8 ou 9, dans lequel la valeur de signification statistique est une valeur R2, et dans lequel le seuil est d'environ 0,90 ou plus.

## FIG. 1

## FIG. 2

**FIG. 3    Spike identification and replacement process flowchart**

This flowchart models the Levenberg-Marquardt outlier method
File: LMOM_SpikeDetector.cs
Class: LMOM_SpikeDetector
Function: CorrectSpikes
Lines: 256-555

The equation used to model PCR curves is a double sigmoid:
a + bx + c / (1+Exp(-d*(x-e))) (1+Exp(-f*(x-g)))

Variables Summary

a: 1st parameter of the double sigmoid equation
b: 2nd parameter of the double sigmoid equation
c: 3rd parameter of the double sigmoid equation
d: 4th parameter of the double sigmoid equation
e: 5th parameter of the double sigmoid equation
f: 6th parameter of the double sigmoid equation
g: 7th parameter of the double sigmoid equation
MAPETreshold: Cutoff for the MAPE value of a curve fit to be considered valid
ziTreshold1: Cutoff value for the Z-Value of a point to be considered a spike
ziTreshold2: Cutoff value for the Z-Value of a point to be considered a big spike

502

Set Parameters b,d and f.

Do/ Set (d,f) = (0.1,0.7), (1.0,0.4), (0.35,0.25)
Do/ Set b =0.01 for all inital parameter sets.

These parameters don't depend
on the curve.

504

Set Parameters a and c

Do/ Set a = 3rd lowest Y-Value for every set of initial parameters.
Do/ Set c = 3rd highest Y-Value -a  for the two first sets of initial parameters.
Do/ Set c = 3rd highest Y-Value-a+2 for the last set of initial parameters.

These parameters depend on the
curve and require minimum
calculation.

Set Parameters e and g

506

These parameters depend on the
curve and require more
calculation.

510

Set the LM with the initial
set of parameters.

Run Levenberg-Marquardt

■ Exit/ The LM Returns the fit value of its approximation

[The fit is worse than the best fit]

[The fit is better than the best fit ]

Save the best Parameters

■ Do/ Save the best fit value
Do/ Save the best set of parameters

[All initial parameters have been tried]

[There are intial parameters that haven't been tried]

Set the LM with the next set of parameters

[GOFMethod = 0]

[GOFMethod = 2]

Move the baseline to 1

[GOFMethod = 3]

Get the MAPE value

[GOFMethod = 1]

Get the MAD

[GOF > GOFTreshold]

[GOF < GOFTreshold]

Get Z-Values

ZTreshold1 default value = 3.5
ZTreshold2 default value = 15

[ziTreshold2>MAX(Z-Values)>zi Treshold1]

[MAX(Z-Values) < ziTreshold1]

[MAX(Z-Values)>ziTreshold2]

Count and reccord the big spikes

■ Do/ For every point above the ziTreshold2: reccord its position
Do/ For every point above the ziTreshold2: spikePoints++

Replace spike points with cubic spline approximation

During this phase, only the big spikes are replaced

Set the LM with the initial set of parameters.

**FIG. 3 (cont.)**

Run Levenberg-Marquardt

■ Exit/ The LM Returns the fit value of its approximation

This time, the curve doesn't
contain any big spikes any more.
The result should therefore be
more accurate

[The fit is worse than the best fit]

[The fit is better than the best fit]

Save the best Parameters

■ Do/ Save the best fit value
Do/ Save the best set of parameters

[All initial parameters have been tried]

[There are intial parameters that
haven't been tried]

Set the LM with the next
set of parameters

Get Z-Values

Count and reccord the small spike points

■ Do/ For every point above the ziTreshold1: reccord its position
Do/ For every point above the ziTreshold1: spikePoints++

Return a bad approximation error code

■ Do/ Create a return array result curve of length 1
Do/ Fill both return arrays with -1 error code
Do/Create a return array spikePoints of length 1
Exit/ Return the result curve and spikePoints arrays

Replace spike points with cubic spline approximation

■ Exit/ Returns a corrected curve as result curve
Exit/ Returns an array of the positions of the corrected spikes

The Curve contains no spikes.

■ Exit/ Return a hard copy of the curve as result curve
Exit/ Return an empty array as SpikePoints

**FIG. 3 (cont.)**

**FIG. 4**   **Double sigmoid decomposition**

a   constant term
b   linear term
s1   sigmoid 1
s2   sigmoid 2
s   resultant double sigmoid

**FIG. 5**   **The parameters of a sigmoid**

**FIG. 6**      **Initial parameters shapes**

| | d | f |
|---|------|------|
| 1 | 0.1 | 0.7 |
| 2 | 1.0 | 0.4 |
| 3 | 0.35 | 0.25 |

# FIG. 7     Parameter e and g calculation flowchart

This flowchart models the way the double-sigmoid parameters e and g are calculated
File: LMOMSpike_Detecor.cs
Class: LMOM_SpikeDetector
Function: CorrectSpikes
Lines: 302-340

Variables Summary:

e: current value of the parameter e
g: current value of the parameter g
mean: value of the mean of the y-values of the curve
numCycles: the number of cycles of the curve
toCorrect: an array containing the y-values of the curve
preSpikeLoops: stores how many candidates for e and g have been tested

Get the mean of the
fluorescence.

Do/ mean = Mean(toCorrect)

Set e = 5

[e > numCycles - 5]

[e < numCycles - 5]

[toCorrect[e]<mean]

[toCorrect[e] > mean]

Increase the value of the
Spike Checking loops by
one.

Do/ preSpikeLoops++

[The Derivatives around e don't have
sign change]

[The derivatives around e have a sign
change]

Increase e

Do/e++

Return e = g = 2/3
Number of Cycles

Do/ e=g=2/3*numCycles

Return e = g = e - the
number of Spike Checking
loops .

Do/e=g=e-preSpikeLoops

The reason we substract the
number of loops that were
candidates, is that if the curve has
a lot of noise, it will still give us a
good approximate value.

## FIG. 8     Levenberg-Marquardt Process flowchart

This flowchart models the Levenberg-Marquardt algorithm
File: LevenMarNR.cs
Class: LevenMarNR
Function: CalculateParams
Lines: 69-215

Variables Summary

da: vector containing the increment for the new parameter set
diff: the difference between the sum of absolute errors of two consecutive iterations
iteration: the number of iteration done
lamda: the damping factor of the Levenberg-Marquardt method
maxIteration: the maximum number of iteration
oneda: Jacobian^T*Error
temp: the Hessian Matrix, augmented during the "Augment the Hessian Matrix" activity

Set the initial variables

Do/ diff = 2.0
Do/ iteration = 0
Do/ lamda = 0.001

Evaluate the fit of a set of parameters and prepare matrices

The parameters used for this first iterations are the initial parameters previously loaded.

The Matrices prepared during this step are the Hessian Matrix of the fit and the Jacobian Matrix * (y[pred]-y[data])

[iterations>maxIterations || diff < 0.0001 || lamda > 10^20]

maxIteration default value is 100.

[iterations<maxIterations && diff > 0.0001 && lamda < 10^20]

Increment iteration

Do/iteration++

Augment the Hessian Matrix

Do/ Add lamda to the diagonal of temp

Solve the augmented equation:
temp * oneda = da

■ Exit/ Returns da, the solution vector

da is the Jacobian Matrix*(y[pred]-y[data])          ─ ■ \Error during Gauss-Jordan\

temp is the augmented Hessian Matrix.

The equation is solved using the
Gauss-Jordan Method

Increment Parameters

■ Do/ Add da to the current parameters

The previous set of parameters is
stored in a different array.

Evaluate the fit of a set
of parameters and
prepare matrices

[newFit < oldFit && -1 < parameter          [newFit > oldFit || -1 > parameter d,f
d,f < 1.1]                                   || parameter d,f > 1.1]

Test wether the new set of
parameters fits the data better.

Keep new set of parameters                      Throw away new set of
                                                    parameters
■ Do/ Decrease lamda: lamda = 0.1*lamda
■ Do/ update the best fit value                 ■ Do/ Increase lamda: lamda = 10*lamda
■ Do/ update the best set of parameters
■ Do/ update the difference between two iterations, diff = oldFit-newFit

Return the current set of parameters                  Return the best parameters so far

■ Exit/ The last set of parameters is returned        ■ Exit/ Its fit in terms of sum of absolute error is returned
■ Exit/ The value of the absolute sum of errors of the best fit is returned    ■ Exit/ The best set of parameters calculated before the error is returned

**FIG. 8 (cont.)**

33

**FIG. 9**

| Acquire Data set | — 910 |

↓

| Approximate Curve Fit | — 920 |

↓

| Normalize Data | — 930 |

↓

| Apply Curvature Determination Process to Determine Elbow Value | — 940 |

↓

| Return Elbow value | — 950 |

↓

| Display Data | — 960 |

## FIG. 10a   PCR data

## FIG. 10b   Curvature

## FIG. 11   Radius of Curvature

## FIG. 12a   Raw Data

| Cycle Number | Signal |
|:---:|:---:|
| 1 | 1.47 |
| 2 | 1.52 |
| 3 | 1.52 |
| 4 | 1.53 |
| 5 | 1.52 |
| 6 | 1.51 |
| 7 | 1.54 |
| 8 | 1.52 |
| 9 | 1.55 |
| 10 | 1.51 |
| 11 | 1.60 |
| 12 | 1.57 |
| 13 | 1.60 |
| 14 | 1.59 |
| 15 | 1.57 |
| 16 | 1.64 |
| 17 | 1.63 |
| 18 | 1.63 |
| 19 | 1.68 |
| 20 | 1.60 |
| 21 | 1.69 |
| 22 | 1.70 |
| 23 | 1.63 |
| 24 | 1.76 |
| 25 | 1.71 |
| 26 | 1.74 |
| 27 | 1.70 |
| 28 | 1.68 |
| 29 | 1.81 |
| 30 | 1.81 |
| 31 | 1.82 |
| 32 | 1.83 |
| 33 | 1.86 |
| 34 | 2.05 |
| 35 | 2.47 |
| 36 | 2.94 |
| 37 | 3.43 |
| 38 | 3.93 |
| 39 | 4.32 |
| 40 | 4.69 |
| 41 | 4.94 |
| 42 | 5.27 |
| 43 | 5.59 |
| 44 | 5.88 |
| 45 | 6.12 |
| 46 | 6.43 |
| 47 | 6.78 |
| 48 | 7.08 |
| 49 | 7.35 |
| 50 | 7.67 |
| 51 | 7.92 |
| 52 | 8.21 |
| 53 | 8.54 |
| 54 | 8.86 |
| 55 | 9.11 |

| Cycle Number | Signal |
|:---:|:---:|
| 56 | 9.36 |
| 57 | 9.65 |
| 58 | 9.89 |
| 59 | 10.15 |
| 60 | 10.44 |

FIG. 12b    Raw Data

FIG. 13    Raw Data and Double Sigmoid Fit

**FIG. 14**      **Normalized Data and Double Sigmoid Fit**

**FIG. 15**      **Curvature Plot**

**FIG. 16**      **Radius of Curvature**

**FIG. 17      Raw Data**

**FIG. 18      Baseline Subtraction and Double Sigmoid**

**FIG. 19      Curvature Plot**

**FIG. 20    Raw Data Set**

**FIG. 21**

**FIG. 22**

**FIG. 23**

## FIG. 24

thermocycler device

external storage medium

storage medium

display device

kinetic PCR analysis module

PCR data set
(transfer via network connection or direct connection)

intelligence module

processing device

RAM

ROM

storage medium

Input device (mouse, keyboard, interface, port)

external storage medium

computer system

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9746707 A
- WO 9746712 A
- WO 9746714 A
- EP 1798650 A

- EP 1804172 A
- EP 1335028 A
- US 20070148632 A

**Non-patent literature cited in the description**

- **GIBSON et al.** *Genome Research,* vol. 6, 995-1001
- **BIECHE et al.** *Cancer Research,* 1999, vol. 59, 2759-2765
- **WANG ; SHA-SHA et al.** Homogeneous Real-Time Detection of Single-Nucleotide Polymorphisms by Strand Displacement Amplification on the BD ProbeTec ET System. *Clin Chem,* 2003, vol. 49 (10), 1599

- **WEUSTEN ; JOS J.A.M. et al.** Principles of Quantitation of Viral Loads Using Nucleic Acid Sequence-Based Amplification in Combination With Homogeneous Detection Using Molecular Beacons. *Nucleic Acids Research,* 2002, vol. 30 (6), 26